# EUROPÄISCHE PATENTSCHRIFT

(11) **EP 3 016 952 B1**
(45) Veröffentlichungstag und Bekanntmachung des Hinweises auf die Patenterteilung: **15.05.2019**
(21) Anmeldenummer: 14733077.3
(22) Anmeldetag: 03.06.2014
(51) Int. Cl.: C07D 471/06, C07D 471/20, C07D 471/22, C07D 491/20, C07D 491/22, C07D 495/20, C07D 495/22, C09K 11/06, H05B 33/00

(54) **SPIROKONDENSIERTE LACTAMVERBINDUNGEN FÜR ORGANISCHE ELEKTROLUMINESZENZVORRICHTUNGEN**
SPIRO-CONDENSED LACTAM COMPOUNDS FOR ORGANIC ELECTROLUMINESCENT DEVICES
COMPOSÉS DE LACTAME SPIROCONDENSÉS POUR DES DISPOSITIFS ÉLECTROLUMINESCENTS ORGANIQUES

(30) Priorität: 02.07.2013 EP 13003343
(43) Veröffentlichungstag der Anmeldung: 11.05.2016
(73) Patentinhaber: Merck Patent GmbH, 64293 Darmstadt (DE)
(72) Erfinder: PARHAM, Amir Hossain, 60486 Frankfurt am Main (DE); PFLUMM, Christof, 64291 Darmstadt (DE); EBERLE, Thomas, 76829 Landau (DE); JATSCH, Anja, 60489 Frankfurt am Main (DE); MONTENEGRO, Elvira, 69469 Weinheim (DE); KROEBER, Jonas Valentin, D-60311 Frankfurt am Main (DE); LINGE, Rouven, 64291 Darmstadt (DE)
(86) Internationale Anmeldenummer: PCT/EP2014/001497
(87) Internationale Veröffentlichungsnummer: WO 2015/000542

(56) Entgegenhaltungen:
- WO-A1-2013/064206
- DE-A1-102010 012 738
- GÁBOR MÉHES ET AL: "Enhanced Electroluminescence Efficiency in a Spiro-Acridine Derivative through Thermally Activated Delayed Fluorescence", ANGEWANDTE CHEMIE INTERNATIONAL EDITION, Bd. 51, Nr. 45, 8. Oktober 2012 (2012-10-08), Seiten 11311-11315, XP055115730, ISSN: 1433-7851, DOI: 10.1002/anie.201206289

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, die diese Materialien enthalten.

In organischen Elektrolumineszenzvorrichtungen (OLEDs) werden als emittierende Materialien zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Dies gilt insbesondere auch für OLEDs, welche im kürzerwelligen Bereich, beispielsweise grün, emittieren.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Lactame (z. B. gemäß WO 2011/116865, WO 2011/137951 oder WO 2013/064206) als Matrixmaterialien für phosphoreszierende Emitter verwendet. Allerdings besteht bei Verwendung dieser Matrixmaterialien ebenso wie bei anderen Matrixmaterialien noch Verbesserungsbedarf, insbesondere in Bezug auf die Effizienz und die Lebensdauer der Vorrichtung.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Lochtransport-/ Elektronenblockiermaterial bzw. Exzitonenblockiermaterial oder als Elektronentransport- bzw. Lochblockiermaterial. Insbesondere ist es die Aufgabe der vorliegenden Erfindung, Matrixmaterialien bereitzustellen, welche sich für grün, gelb und rot phosphoreszierende OLEDs und je nach genauer Struktur auch für blau phosphoreszierende OLEDs eignen.

Überraschend wurde gefunden, dass bestimmte, unten näher beschriebene Verbindungen diese Aufgabe lösen und zu sehr guten Eigenschaften der organischen Elektrolumineszenzvorrichtung führen, insbesondere hinsichtlich der Lebensdauer, der Effizienz und der Betriebsspannung. Dies gilt insbesondere für rot, gelb und grün phosphoreszierende Elektrolumineszenzvorrichtungen, vor allem bei Einsatz der erfindungsgemäßen Verbindungen als Matrixmaterial. Die Materialien zeichnen sich weiterhin durch hohe Oxidationsstabilität in Lösung sowie durch eine hohe Temperaturstabilität aus. Diese Materialien sowie elektronische Vorrichtungen, insbesondere organische Elektrolumineszenzvorrichtungen, welche derartige Materialien enthalten, sind daher der Gegenstand der vorliegenden Erfindung.

Gegenstand der vorliegenden Erfindung ist eine Verbindung gemäß der folgenden Formel (1), 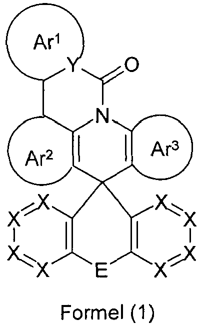 wobei für die verwendeten Symbole und Indizes gilt:
- X: ist bei jedem Auftreten gleich oder verschieden CR oder N;
- Y: ist C, wenn Ar¹ eine 6-Ring-Aryl- oder Heteroarylgruppe darstellt, bzw. ist C oder N, wenn Ar¹ eine 5-Ring-Heteroarylgruppe darstellt;
- E: ist NR, CR₂, O, S oder C=O;
- Ar¹: ist zusammen mit der Gruppe Y und dem explizit dargestellten Kohlenstoffatom eine Gruppe der Formel (2), (3), (4), (5) oder (6), 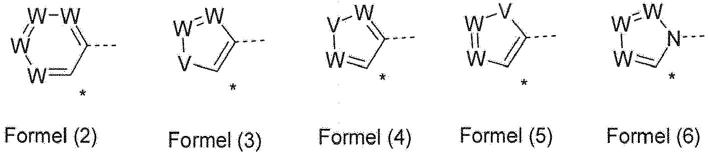 wobei die gestrichelte Bindung die Verknüpfung mit der Carbonylgruppe des Lactams andeutet, * die Position der Verknüpfung mit Ar² andeutet und weiterhin gilt:
W ist gleich oder verschieden bei jedem Auftreten CR oder N;
V ist NR, O oder S;
- Ar²: ist zusammen mit den explizit dargestellten Kohlenstoffatomen eine Gruppe gemäß einer der Formeln (9), (10) oder (11); 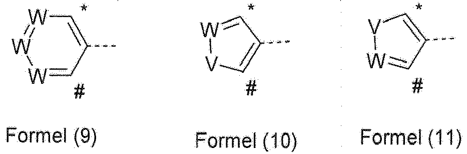
wobei die gestrichelte Bindung die Verknüpfung mit N andeutet, # die Position der Verknüpfung mit dem Spiro-Kohlenstoffatom andeutet, * die Verknüpfung mit Ar¹ andeutet und W und V die oben genannten Bedeutungen aufweisen;
- Ar³: ist zusammen mit den explizit dargestellten Kohlenstoffatomen eine Gruppe gemäß einer der Formeln (12), (13), (14) oder (15), 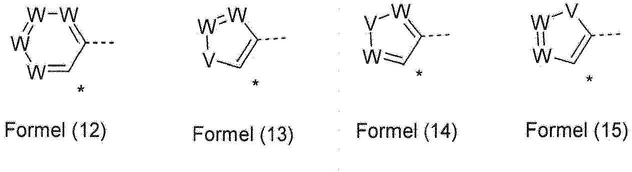 wobei die gestrichelte Bindung die Verknüpfung mit N andeutet, * die Verknüpfung mit dem Spiro-Kohlenstoffatom andeutet und W und V die oben genannten Bedeutungen aufweisen;
- R: ist bei jedem Auftreten gleich oder verschieden ausgewählt aus der Gruppe bestehend aus H, D, F, Cl, Br, CN, N(Ar⁴)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R¹ substituiert sein kann, einer
Aryloxy- oder Heteroaryloxygruppe mit 5 bis 30 aromatischen Ringatomen, die mit einem oder mehreren Resten R¹ substituiert sein kann, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann;
- R¹: ist bei jedem Auftreten gleich oder verschieden ausgewählt aus der Gruppe bestehend aus H, D, F, Cl, Br, CN, einer geradkettigen Alkyl- oder Alkoxygruppe mit 1 bis 40 C-Atomen oder einer verzweigten oder cyclischen Alkyl- oder Alkoxygruppe mit 3 bis 40 C-Atomen oder einer Alkenylgruppe mit 2 bis 40 C-Atomen, die jeweils mit einem oder mehreren Resten R² substituiert sein kann, wobei ein oder mehrere H-Atome durch D oder F ersetzt sein können, einem aromatischen oder heteroaromatischen Ringsystem mit 5 bis 60 aromatischen Ringatomen, das jeweils mit einem oder mehreren Resten R² substituiert sein kann;
- Ar⁴: ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar⁴, welche an dasselbe N-Atom binden, auch durch eine Einfachbindung oder eine Brücke, ausgewählt aus N(R²), C(R²)₂ oder O, miteinander verbrückt sein;
- R²: ist ausgewählt aus der Gruppe bestehend aus H, D, F, CN oder einem aliphatischem Kohlenwasserstoffrest mit 1 bis 20 C-Atomen.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

In einer bevorzugten Ausführungsform der Erfindung steht X für CR oder N, wobei maximal eine Gruppe X pro Cyclus für N steht. Ganz besonders bevorzugt steht X für CR.

In einer weiteren bevorzugten Ausführungsform der Erfindung steht E für NR, wobei R ungleich H oder D ist, CR₂ oder O.

In einer besonders bevorzugten Ausführungsform der Erfindung gilt für Verbindungen der Formel (1):
- X: ist gleich oder verschieden bei jedem Auftreten CR;
- E: ist gleich oder verschieden bei jedem Auftreten NR, wobei R ungleich H oder D ist, CR₂ oder O;
- Ar¹: ist ausgewählt aus den Gruppen der oben genannten Formeln (2), (3), (4), (5) oder (6);
- Ar²: ist ausgewählt aus den Gruppen der oben genannten Formeln (9), (10) oder (11);
- Ar³: ist ausgewählt aus den Gruppen der oben genannten Formeln (12), (13), (14) oder (15).

Dabei können die oben genannten bevorzugten Gruppen Ar¹, Ar² und Ar³ beliebig miteinander kombiniert werden. Geeignete Kombinationen sind also die Folgenden:

| Ar¹ | Ar² | Ar³ |
|---|---|---|
| Formel (2) | Formel (9) | Formel (12) |
| Formel (2) | Formel (9) | Formel (13) |
| Formel (2) | Formel (9) | Formel (14) |
| Formel (2) | Formel (9) | Formel (15) |
| Formel (2) | Formel (10) | Formel (12) |
| Formel (2) | Formel (10) | Formel (13) |
| Formel (2) | Formel (10) | Formel (14) |
| Formel (2) | Formel (10) | Formel (15) |
| Formel (2) | Formel (11) | Formel (12) |
| Formel (2) | Formel (11) | Formel (13) |
| Formel (2) | Formel (11) | Formel (14) |
| Formel (2) | Formel (11) | Formel (15) |
| Formel (2) | Formel (12) | Formel (12) |
| Formel (2) | Formel (12) | Formel (13) |
| Formel (2) | Formel (12) | Formel (14) |
| Formel (2) | Formel (12) | Formel (15) |
| Formel (3) | Formel (9) | Formel (12) |
| Formel (3) | Formel (9) | Formel (13) |
| Formel (3) | Formel (9) | Formel (14) |
| Formel (3) | Formel (9) | Formel (15) |
| Formel (3) | Formel (10) | Formel (12) |
| Formel (3) | Formel (10) | Formel (13) |
| Formel (3) | Formel (10) | Formel (14) |
| Formel (3) | Formel (10) | Formel (15) |
| Formel (3) | Formel (11) | Formel (12) |
| Formel (3) | Formel (11) | Formel (13) |
| Formel (3) | Formel (11) | Formel (14) |
| Formel (3) | Formel (11) | Formel (15) |
| Formel (3) | Formel (12) | Formel (12) |
| Formel (3) | Formel (12) | Formel (13) |
| Formel (3) | Formel (12) | Formel (14) |
| Formel (3) | Formel (12) | Formel (15) |
| Formel (4) | Formel (9) | Formel (12) |
| Formel (4) | Formel (9) | Formel (13) |
| Formel (4) | Formel (9) | Formel (14) |
| Formel (4) | Formel (9) | Formel (15) |
| Formel (4) | Formel (10) | Formel (12) |
| Formel (4) | Formel (10) | Formel (13) |
| Formel (4) | Formel (10) | Formel (14) |
| Formel (4) | Formel (10) | Formel (15) |
| Formel (4) | Formel (11) | Formel (12) |
| Formel (4) | Formel (11) | Formel (13) |
| Formel (4) | Formel (11) | Formel (14) |
| Formel (4) | Formel (11) | Formel (15) |
| Formel (4) | Formel (12) | Formel (12) |
| Formel (4) | Formel (12) | Formel (13) |
| Formel (4) | Formel (12) | Formel (14) |
| Formel (4) | Formel (12) | Formel (15) |
| Formel (5) | Formel (9) | Formel (12) |
| Formel (5) | Formel (9) | Formel (13) |
| Formel (5) | Formel (9) | Formel (14) |
| Formel (5) | Formel (9) | Formel (15) |
| Formel (5) | Formel (10) | Formel (12) |
| Formel (5) | Formel (10) | Formel (13) |
| Formel (5) | Formel (10) | Formel (14) |
| Formel (5) | Formel (10) | Formel (15) |
| Formel (5) | Formel (11) | Formel (12) |
| Formel (5) | Formel (11) | Formel (13) |
| Formel (5) | Formel (11) | Formel (14) |
| Formel (5) | Formel (11) | Formel (15) |
| Formel (5) | Formel (12) | Formel (12) |
| Formel (5) | Formel (12) | Formel (13) |
| Formel (5) | Formel (12) | Formel (14) |
| Formel (5) | Formel (12) | Formel (15) |
| Formel (6) | Formel (9) | Formel (12) |
| Formel (6) | Formel (9) | Formel (13) |
| Formel (6) | Formel (9) | Formel (14) |
| Formel (6) | Formel (9) | Formel (15) |
| Formel (6) | Formel (10) | Formel (12) |
| Formel (6) | Formel (10) | Formel (13) |
| Formel (6) | Formel (10) | Formel (14) |
| Formel (6) | Formel (10) | Formel (15) |
| Formel (6) | Formel (11) | Formel (12) |
| Formel (6) | Formel (11) | Formel (13) |
| Formel (6) | Formel (11) | Formel (14) |
| Formel (6) | Formel (11) | Formel (15) |
| Formel (6) | Formel (12) | Formel (12) |
| Formel (6) | Formel (12) | Formel (13) |
| Formel (6) | Formel (12) | Formel (14) |
| Formel (6) | Formel (12) | Formel (15) |

Besonders bevorzugt stehen mindestens zwei der Gruppen Ar¹, Ar² und Ar³ für eine 6-Ring-Aryl- oder eine 6-Ring-Heteroarylgruppe. Besonders bevorzugt steht also Ar¹ für eine Gruppe der Formel (2) und gleichzeitig steht Ar² für eine Gruppe der Formel (9), oder Ar¹ steht für eine Gruppe der Formel (2) und gleichzeitig steht Ar³ für eine Gruppe der Formel (12), oder Ar² steht für eine Gruppe der Formel (9) und gleichzeitig steht Ar³ für eine Gruppe der Formel (12).

Besonders bevorzugte Ausführungsformen der Erfindung sind daher die Verbindungen der folgenden Formeln (16) bis (25), 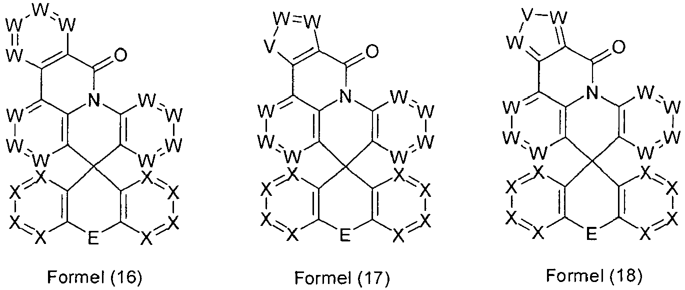 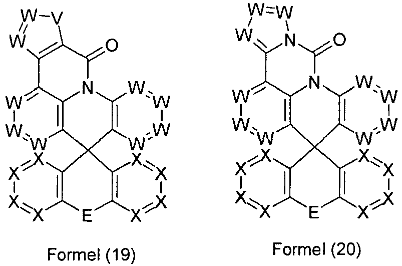 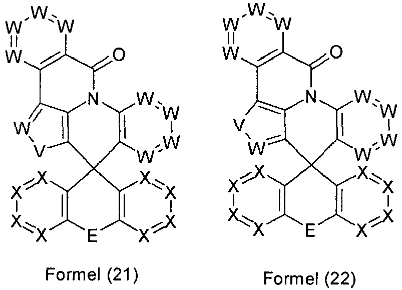 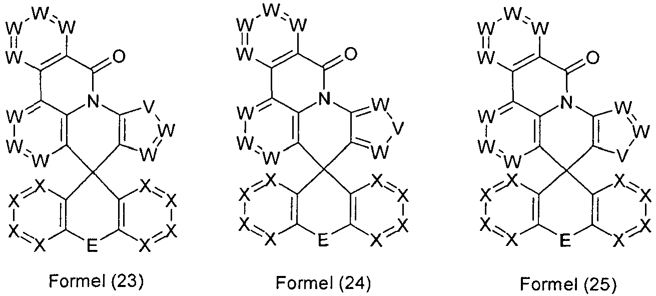 wobei die verwendeten Symbole die oben genannten Bedeutungen aufweisen.

Wie oben bereits ausgeführt, können auch in den Verbindungen der Formeln (16) bis (25) zwei benachbarte Gruppen W für eine Gruppe der oben genannten Formel (7) oder (8) stehen.

In einer weiteren bevorzugten Ausführungsform der Verbindungen gemäß Formel (16) bis (25) steht pro Cyclus insgesamt maximal ein Symbol W für N, und die verbleibenden Symbole W, die nicht für eine Gruppe der Formel (7) oder (8) stehen, stehen für CR. In einer besonders bevorzugten Ausführungsform der Erfindung stehen alle Symbole W, die nicht für eine Gruppe der Formel (7) oder (8) stehen, für CR. Weiterhin stehen besonders bevorzugt alle Symbole X für CR. Besonders bevorzugt sind daher die Verbindungen gemäß den folgenden Formeln (16a) bis (25a), 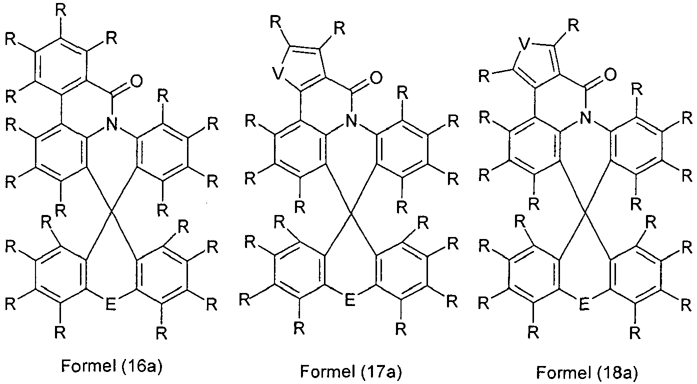 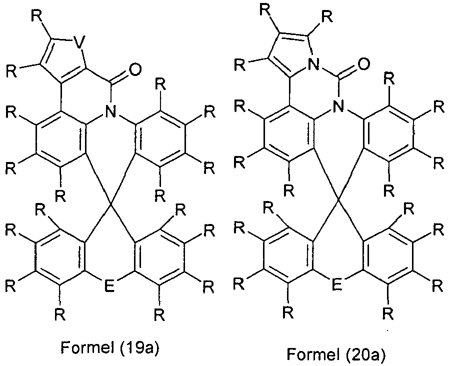 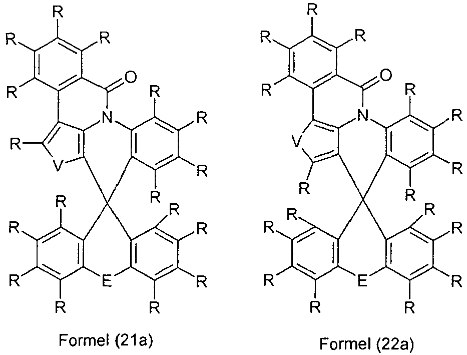 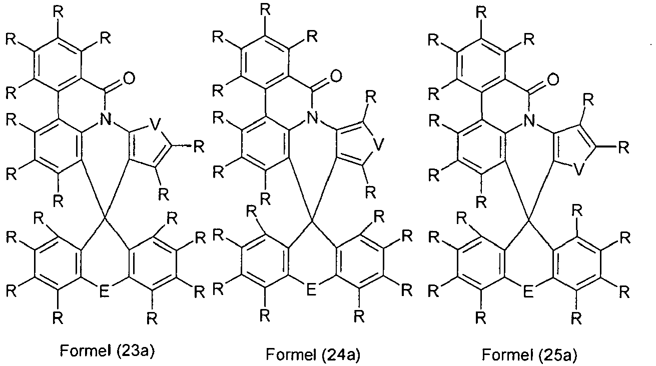 wobei die verwendeten Symbole die oben genannten Bedeutungen aufweisen.

Ganz besonders bevorzugt sind die Verbindungen gemäß den folgenden Formeln (16b) bis (25b), 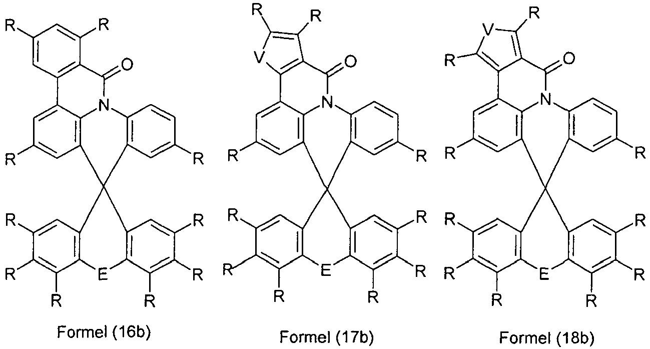 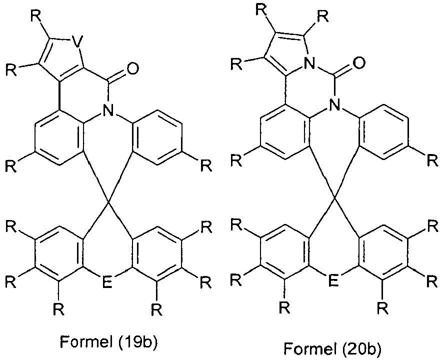 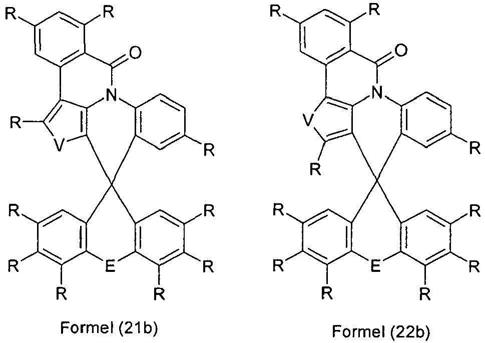 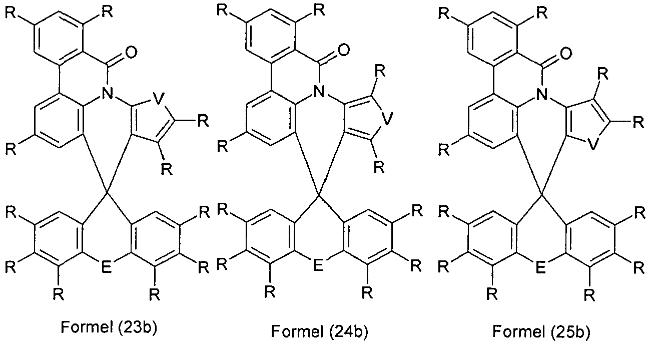 wobei die verwendeten Symbole die oben genannten Bedeutungen aufweisen.

Insbesondere bevorzugt sind die Strukturen der Formeln (16c) bis (25c), 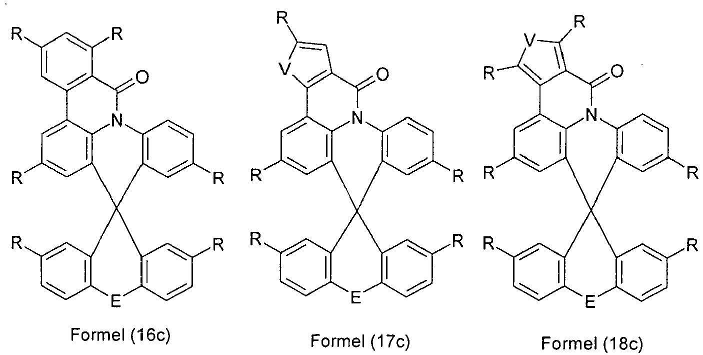 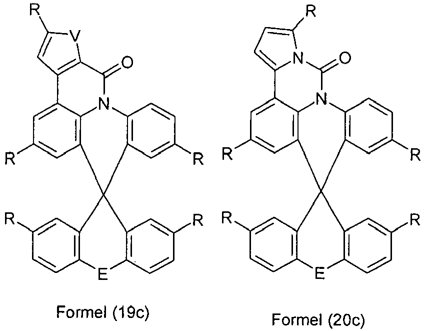 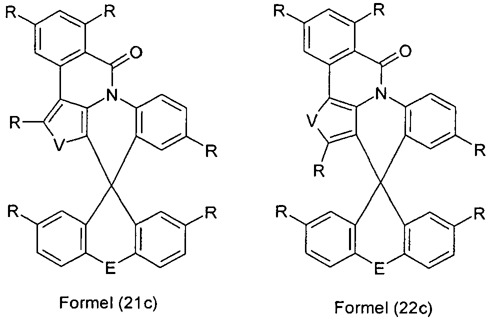 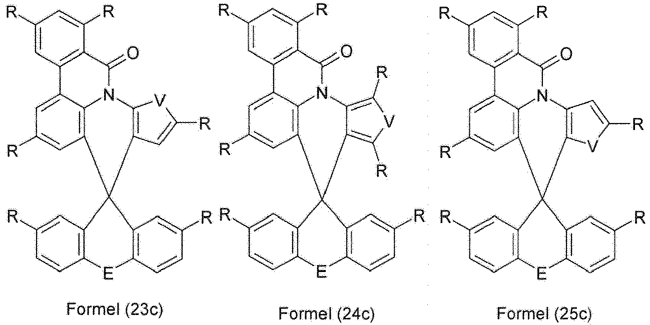 wobei die verwendeten Symbole die oben genannten Bedeutungen aufweisen.

Dabei steht in den Formeln (16) bis (25), (16a) bis (25a), (16b) bis (25b) und (16c) bis (25c) E bevorzugt für CR₂, NR mit R ungleich H, O oder S.

Wenn E für NR steht, so ist R bevorzugt ausgewählt aus aromatischen und heteroaromatischen Ringsystemen mit 5 bis 40 aromatischen Ringatomen, welche mit einem oder mehreren Resten R¹ substituiert sein können. Bevorzugte Reste R, die an das Stickstoffatom gebunden sind, sind Phenyl, ortho-, meta- oder para-Biphenyl, ortho-, meta-, para- oder verzweigtes Terphenyl, ortho-, meta-, para- oder verzweigtes Quaterphenyl, 1-, 2-, 3- oder 4-Fluorenyl, 1-, 2-, 3- oder 4-Spirobifluorenyl, Triazin, 1-, 2-, 3- oder 4-Dibenzofuranyl, 1-, 2-, 3- oder 4-Dibenzothienyl oder 1-, 2-, 3- oder 4-Carbazolyl, die jeweils durch einen oder mehrere Reste R¹ substituiert sein können.

In einer weiteren bevorzugten Ausführungsform der Erfindung ist der Rest R in den oben genannten Formeln, wenn er nicht an den Stickstoff einer Gruppe E bindet, gleich oder verschieden bei jedem Auftreten ausgewählt aus der Gruppe bestehend aus H, D, F, Cl, Br, CN, N(Ar¹)₂, C(=O)Ar¹, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R¹ substituiert sein kann, einer Aryloxy- oder Heteroaryloxygruppe mit 5 bis 30 aromatischen Ringatomen, die mit einem oder mehreren Resten R¹ substituiert sein kann, oder einer Kombination dieser Systeme.

In einer besonders bevorzugten Ausführungsform der Erfindung ist R in den oben genannten Formeln gleich oder verschieden bei jedem Auftreten ausgewählt aus der Gruppe bestehend aus H, F, CN, einer geradkettigen Alkylgruppe mit 1 bis 8 C-Atomen oder einer verzweigten oder cyclischen Alkylgruppe mit 3 bis 8 C-Atomen, die jeweils mit einem oder mehreren Resten R¹ substituiert sein kann, wobei ein oder mehrere H-Atome durch D oder F ersetzt sein können, einem aromatischen oder heteroaromatischen Ringsystem mit 5 bis 18 aromatischen Ringatomen, das jeweils mit einem oder mehreren Resten R¹ substituiert sein kann, oder einer Kombination dieser Systeme.

Geeignete aromatische oder heteroaromatische Ringsysteme sind hier Phenyl, ortho-, meta- oder para-Biphenyl, ortho-, meta-, para- oder verzweigtes Terphenyl, ortho-, meta-, para- oder verzweigtes Quaterphenyl, Carbazol, Indenocarbazol, Indolocarbazol, Triazin, Pyrimidin, 1-, 2-, 3- oder 4-Fluorenyl, 1-, 2-, 3- oder 4-Spirobifluorenyl, 1-, 2-, 3- oder 4-Dibenzofuranyl, 1-, 2-, 3- oder 4-Dibenzothienyl oder 1-, 2-, 3- oder 4-Carbazolyl,die jeweils durch einen oder mehrere Reste R¹ substituiert sein können.

Wenn die Verbindungen der Formel (1) bzw. die bevorzugten Ausführungsformen als Elektronentransportmaterial verwendet werden, kann es bevorzugt sein, wenn mindestens einer der Reste R für ein aromatisches Ringsystem oder ein elektronenarmes heteroaromatisches Ringsystem steht. Elektronenarme Heteroaromaten sind erfindungsgemäß Fünfringheteroaromaten mit mindestens zwei Heteroatomen oder Sechsringheteroaromaten, an die jeweils noch ein oder mehrere aromatische oder heteroaromatische Gruppen ankondensiert sein können.

Wenn die Verbindungen der Formel (1) bzw. die bevorzugten Ausführungsformen als Matrixmaterial für einen phosphoreszierenden Emitter verwendet werden, kann es bevorzugt sein, wenn mindestens einer der Reste R für ein substituiertes oder unsubstituiertes Carbazol, Indenocarbazol oder Indolocarbazol steht, welches jeweils über ein Kohlenstoffatom oder ein Stickstoffatom gebunden sein kann. Weiterhin ist es dann bevorzugt, wenn die Reste R, R¹ und R² keine kondensierten Aryl- oder Heteroarylgruppen enthalten, in denen mehr als zwei aromatische Sechsringe direkt aneinander ankondensiert sind. Besonders bevorzugt enthalten die Reste R, R¹ und R² auch keine kondensierten Aryl- oder Heteroarylgruppen, in denen zwei aromatische Sechsringe direkt aneinander ankondensiert sind.

Dabei haben für Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom.

Die erfindungsgemäßen Verbindungen können auch durch geeignete Substitution, beispielsweise durch längere Alkylgruppen (ca. 4 bis 20 C-Atome), insbesondere verzweigte Alkylgruppen, oder gegebenenfalls substituierte Arylgruppen, beispielsweise Xylyl- oder Mesitylgruppen, oder Oligoarylengruppen, beispielsweise ortho-, meta-, para- oder verzweigten Terphenyl- oder Quaterphenylgruppen, löslich gemacht werden. Solche Verbindungen sind dann in gängigen organischen Lösemitteln, wie beispielsweise Toluol oder Xylol bei Raumtemperatur in ausreichender Konzentration löslich, um die Verbindungen aus Lösung verarbeiten zu können. Diese löslichen Verbindungen eignen sich besonders gut für die Verarbeitung aus Lösung, beispielsweise durch Druckverfahren.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethyleneglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, insbesondere eine Lösung oder Dispersion, enthaltend mindestens eine Verbindung gemäß Formel (1) oder den oben aufgeführten bevorzugten Ausführungsformen und mindestens eine weitere Verbindung, insbesondere ein Lösemittel. Dabei kann die Formulierung aus der Verbindung gemäß Formel (1) und dem oder den Lösemitteln auch noch weitere Verbindungen enthalten, wie beispielsweise Emitter.

Beispiele für bevorzugte Verbindungen gemäß den oben aufgeführten Ausführungsformen sind die Verbindungen der folgenden Strukturen.

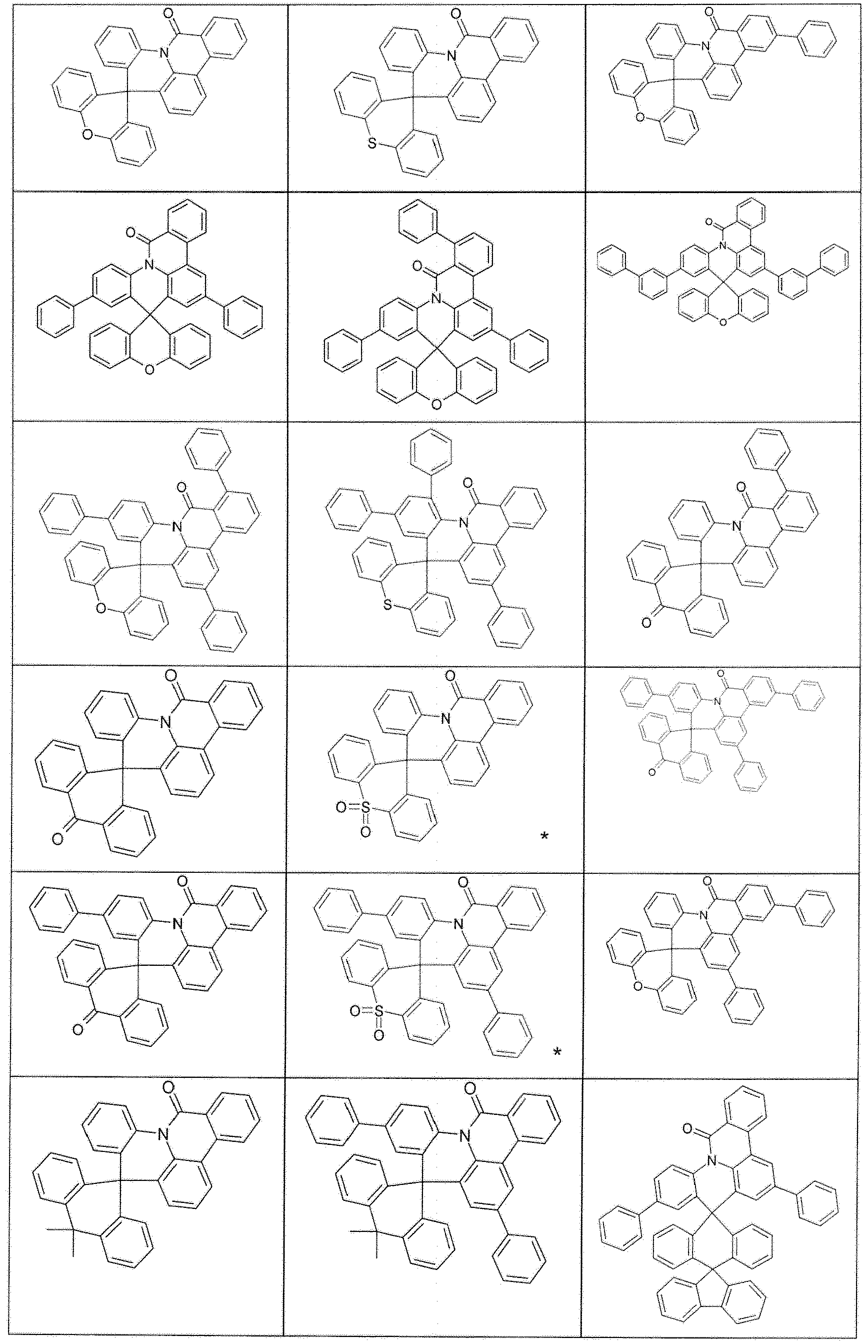

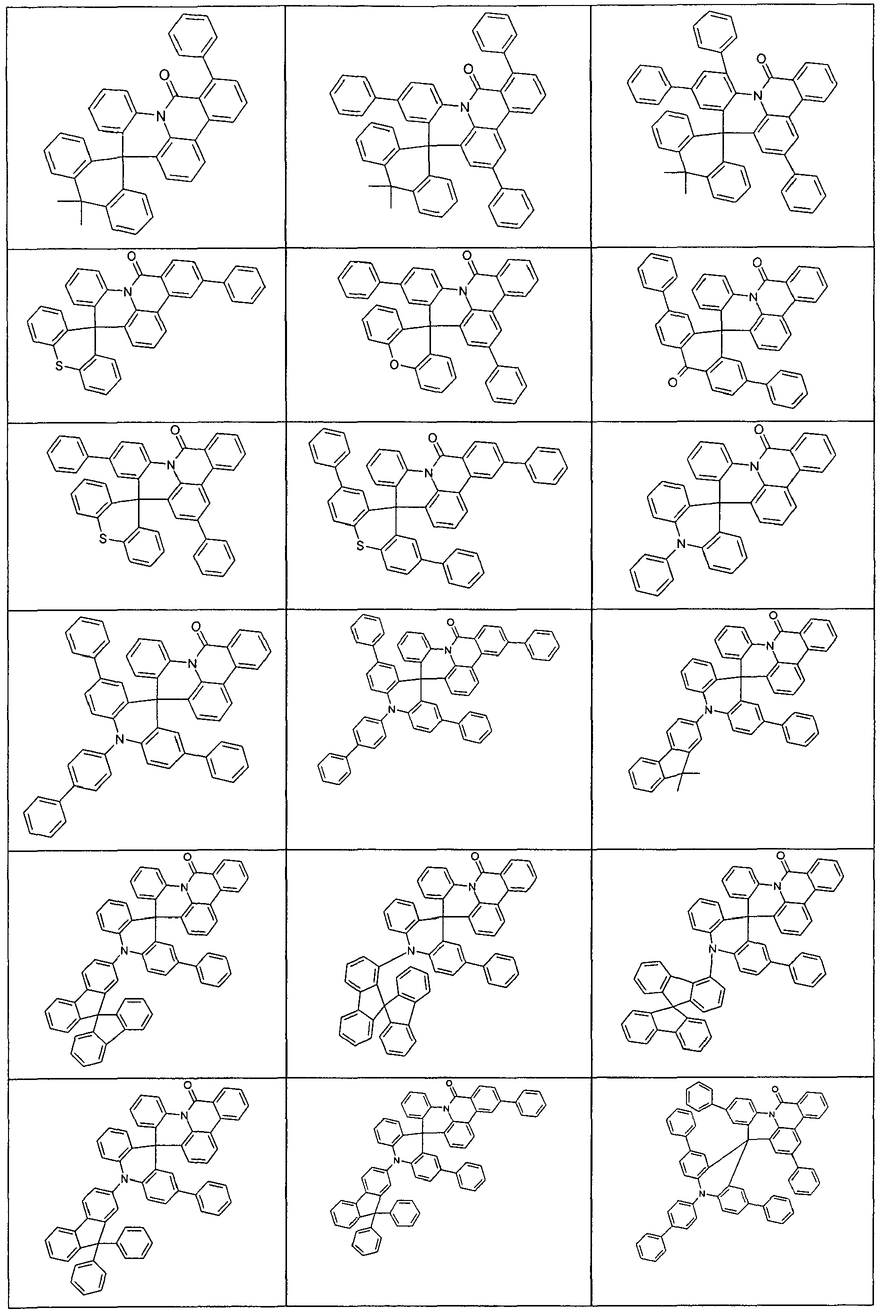

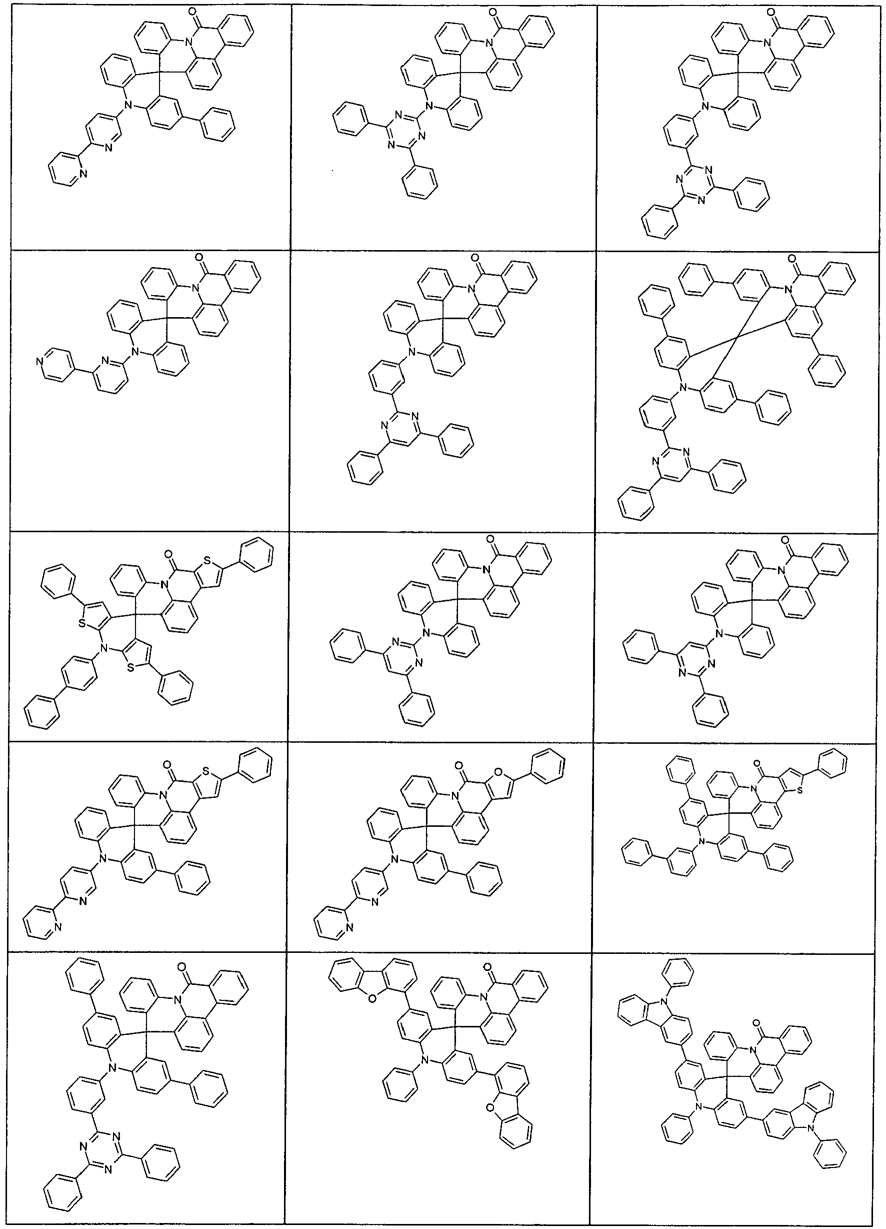

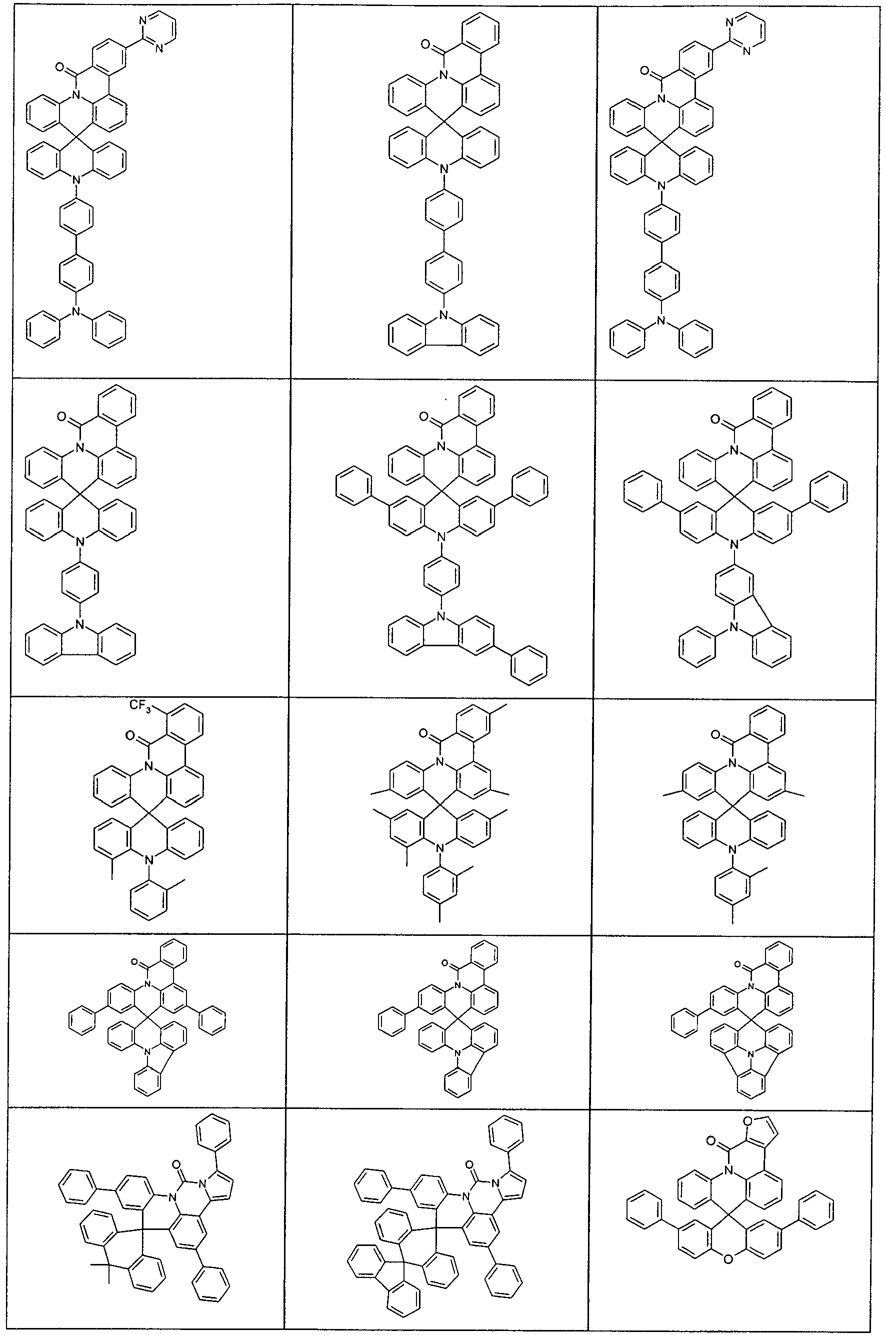

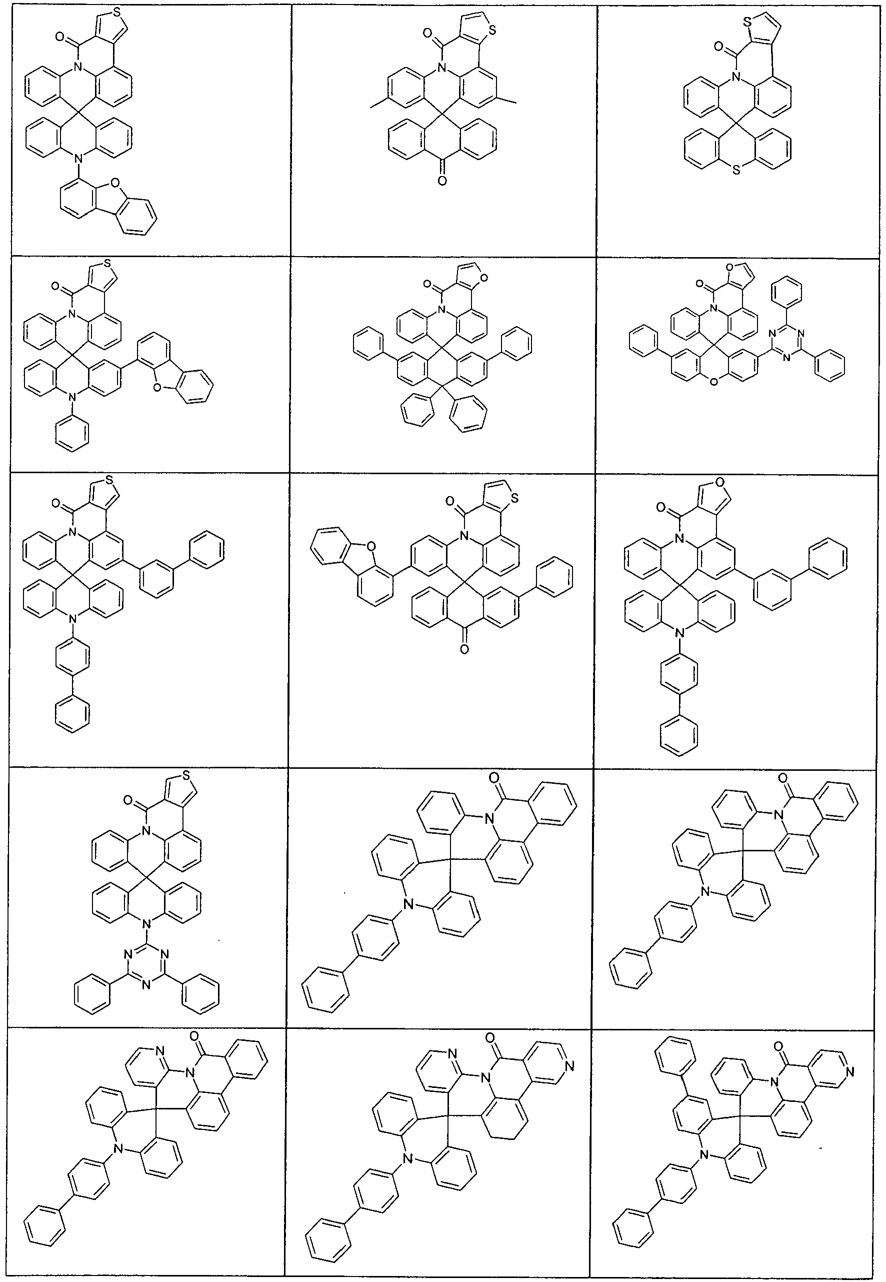

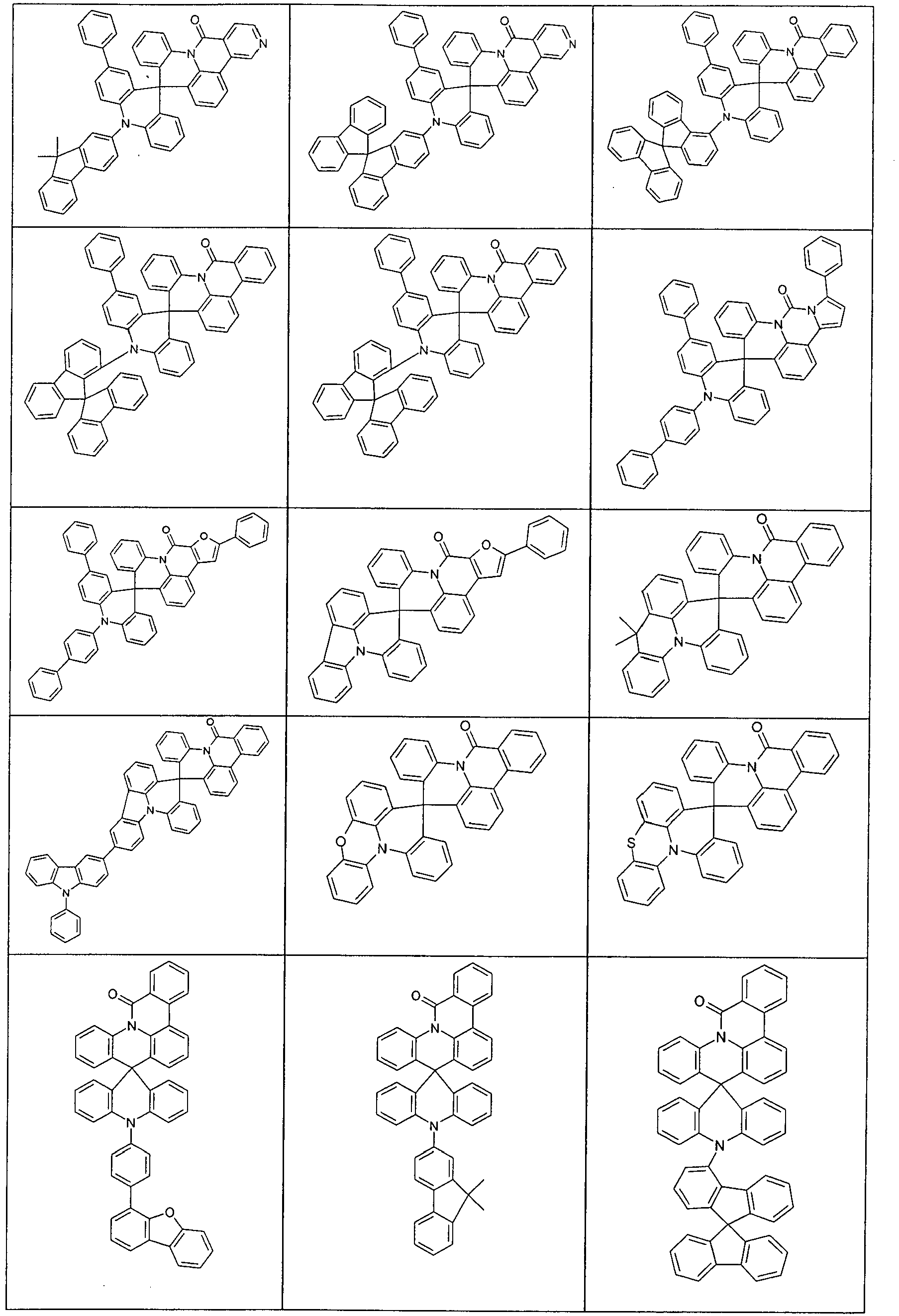

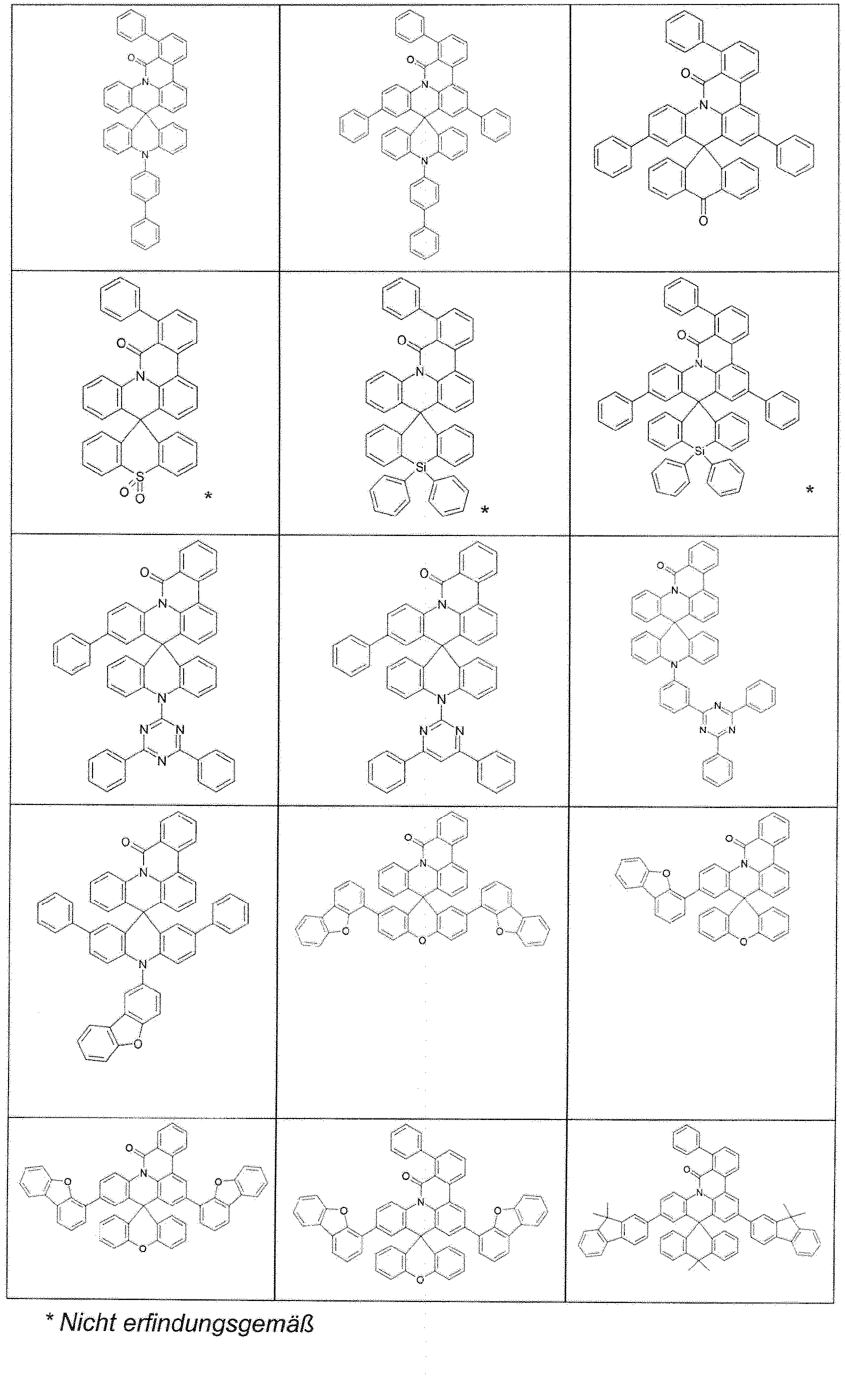

Die Verbindungen der Formel (1) bzw. die bevorzugten Ausführungsformen können nach dem Fachmann bekannten Syntheseschritten dargestellt werden, wie in Schema 1 und 2 schematisch dargestellt.

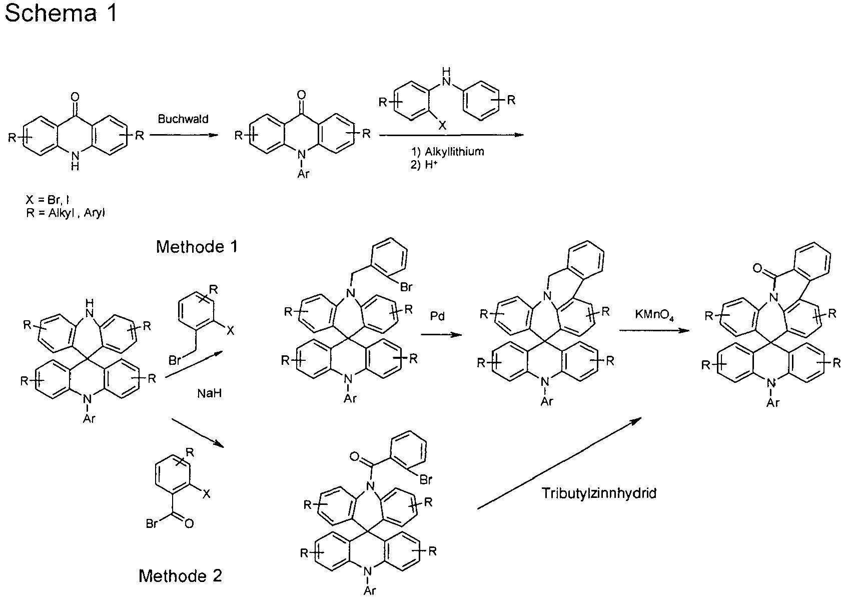

Die funktionalisierten Spiroacridin-Verbindungen stellen den zentralen Baustein für die weitere Funktionalisierung dar, wie in Schema 1 dargestellt. So lassen sich diese funktionalisierten Verbindungen leicht durch Umsetzung der entsprechenden Acridine mit ortho-bromierten Arylaminen durch Lithiierung und säurekatalysierte Cyclisierung herstellen.

Die Spiroacridin-Verbindungen können mit einer Base, beispielsweise NaH, deprotoniert und durch Reaktion mit einem Benzylhalogenid nukleophil substituiert werden. Eine anschließende Palladium-katalysierte Cyclisierung gefolgt von einer Oxidation führt zu den erfindungsgemäßen Verbindungen. Eine weitere Möglichkeit ist die Umsetzung mit einem ortho-halogenierten aromatischen Säurehalogenid und anschließender Cyclisierung, was ebenfalls zu den erfindungsgemäßen Verbindungen führt.

Gand analog können an Stelle des Acridins auch Xanthone, Thoxanthenon oder ein Anthracen-9-on-derivat eingesetzt werden (Schema 2).

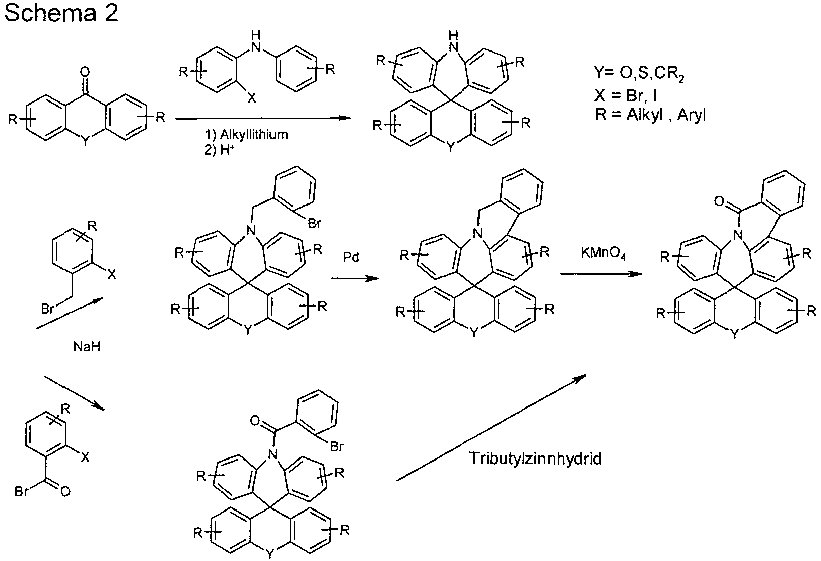

Ein weiterer Gegenstand der vorliegenden Erfindung ist daher ein Verfahren zur Herstellung einer Verbindung gemäß Formel (1), umfassend die Reaktionsschritte:
a) Herstellung einer Spiroverbindung ausgehend von einem Acridin-, Xanthon-, Thioxanthenon- oder Anthracen-9-on-derivat; und
b) Substitution des Stickstoffs gefolgt von Cyclisierung zum Lactam.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei eine oder mehrere Bindungen der erfindungsgemäßen Verbindung zum Polymer, Oligomer oder Dendrimer vorhanden sind. Je nach Verknüpfung der erfindungsgemäßen Verbindung bildet diese daher eine Seitenkette des Oligomers oder Polymers oder ist in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Homopolymere oder Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/22026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten. Außerdem können die Polymere entweder einpolymerisiert oder als Blend eingemischt Triplett-Emitter enthalten. Gerade die Kombination von Einheiten gemäß Formel (1) bzw. der oben ausgeführten bevorzugten Ausführungsformen mit Triplett-Emittern führt zu besonders guten Ergebnissen.

Weiterhin können die Verbindungen gemäß Formel (1) bzw. die oben ausgeführten bevorzugten Ausführungsformen auch weiter funktionalisiert werden und so zu erweiterten Strukturen umgesetzt werden. Hier ist als Beispiel die Umsetzung mit Arylboronsäuren gemäß Suzuki oder mit primären oder sekundären Aminen gemäß Hartwig-Buchwald zu nennen. So können die Verbindungen gemäß Formel (1) bzw. die oben ausgeführten bevorzugten Ausführungsformen auch direkt an phosphoreszierende Metallkomplexe oder auch an andere Metallkomplexe gebunden werden.

Die erfindungsgemäße Verbindung kann in einer elektronischen Vorrichtung eingesetzt werden. Ein weiterer Gegenstand der vorliegenden Erfindung ist daher die Verwendung einer Verbindung gemäß Formel (1) oder den oben aufgeführten bevorzugten Ausführungsformen in einer elektronischen Vorrichtung, insbesondere in einer organischen Elektrolumineszenzvorrichtung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoffsensibilisierten Solarzellen (O-DSSC), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013).

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht, je nach genauer Substitution.

In einer bevorzugten Ausführungsform der Erfindung wird die Verbindung gemäß Formel (1) bzw. die oben ausgeführten bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. die oben ausgeführten bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der Verbindung gemäß Formel (1) bzw. der oben ausgeführten bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. der oben ausgeführten bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bzw. der oben ausgeführten bevorzugten Ausführungsformen als Matrixmaterial für einen phosphoreszierenden Emitter in Kombination mit einem weiteren Matrixmaterial. Dabei kann das weitere Matrixmaterial lochtransportierend oder elektronentransportierend sein, oder es kann sich um ein Matrixmaterial handeln, welches eine große Bandlücke aufweist und somit weder am Loch- noch am Elektronentransport teilnimmt. Besonders geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder der nicht offen gelegten Anmeldung EP 11007693.2 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Wenn die Verbindungen Ir oder Pt enthalten, enthalten sie bevorzugt mindestens einen Liganden, der über ein Kohlenstoffatom und ein Stickstoff- oder ein Sauerstoffatom an das Metall koordiniert.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für geeignete phosphoreszierende Emitter sind die in der folgenden Tabelle aufgeführten Verbindungen.

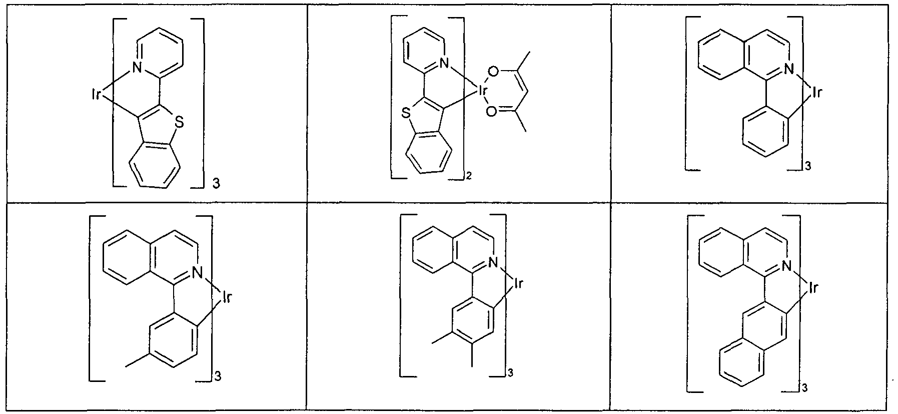

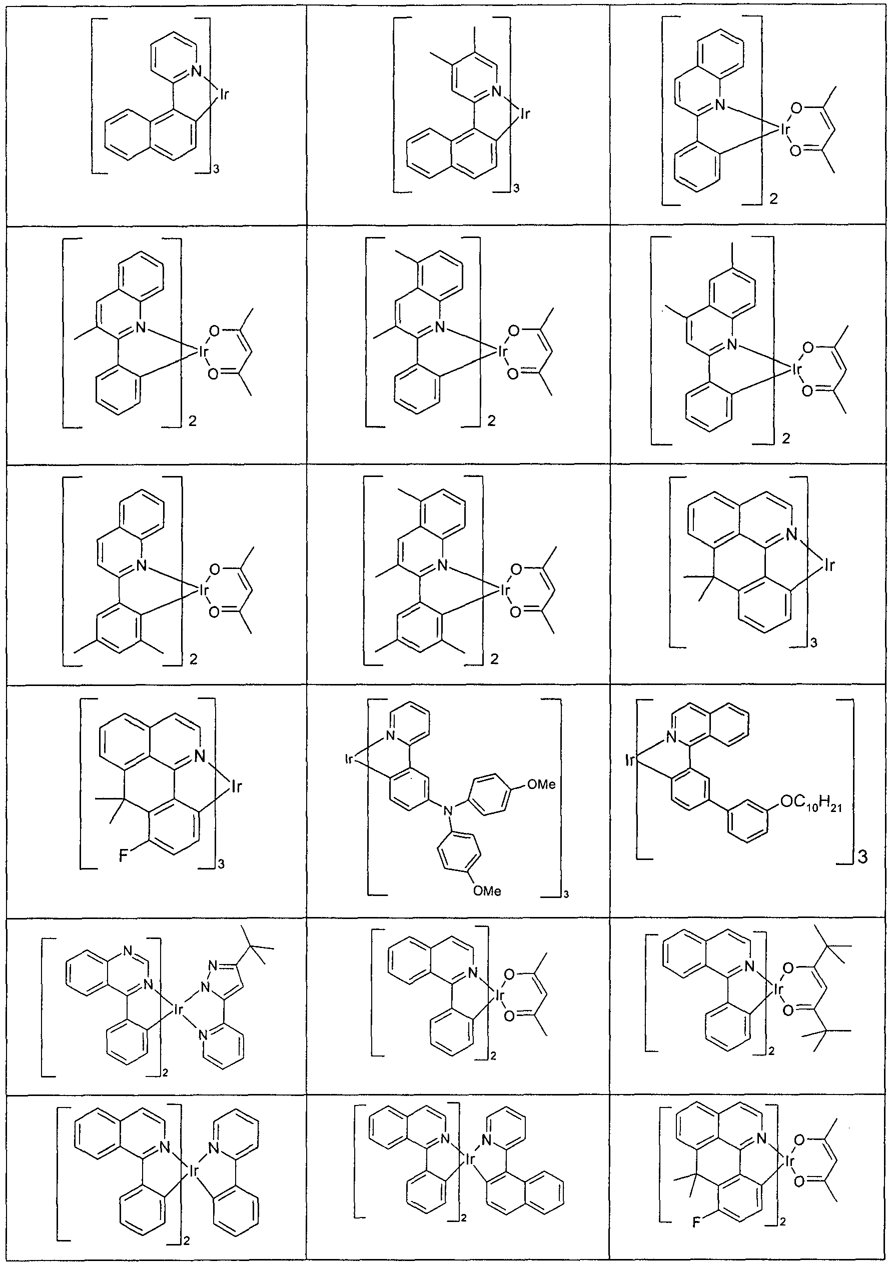

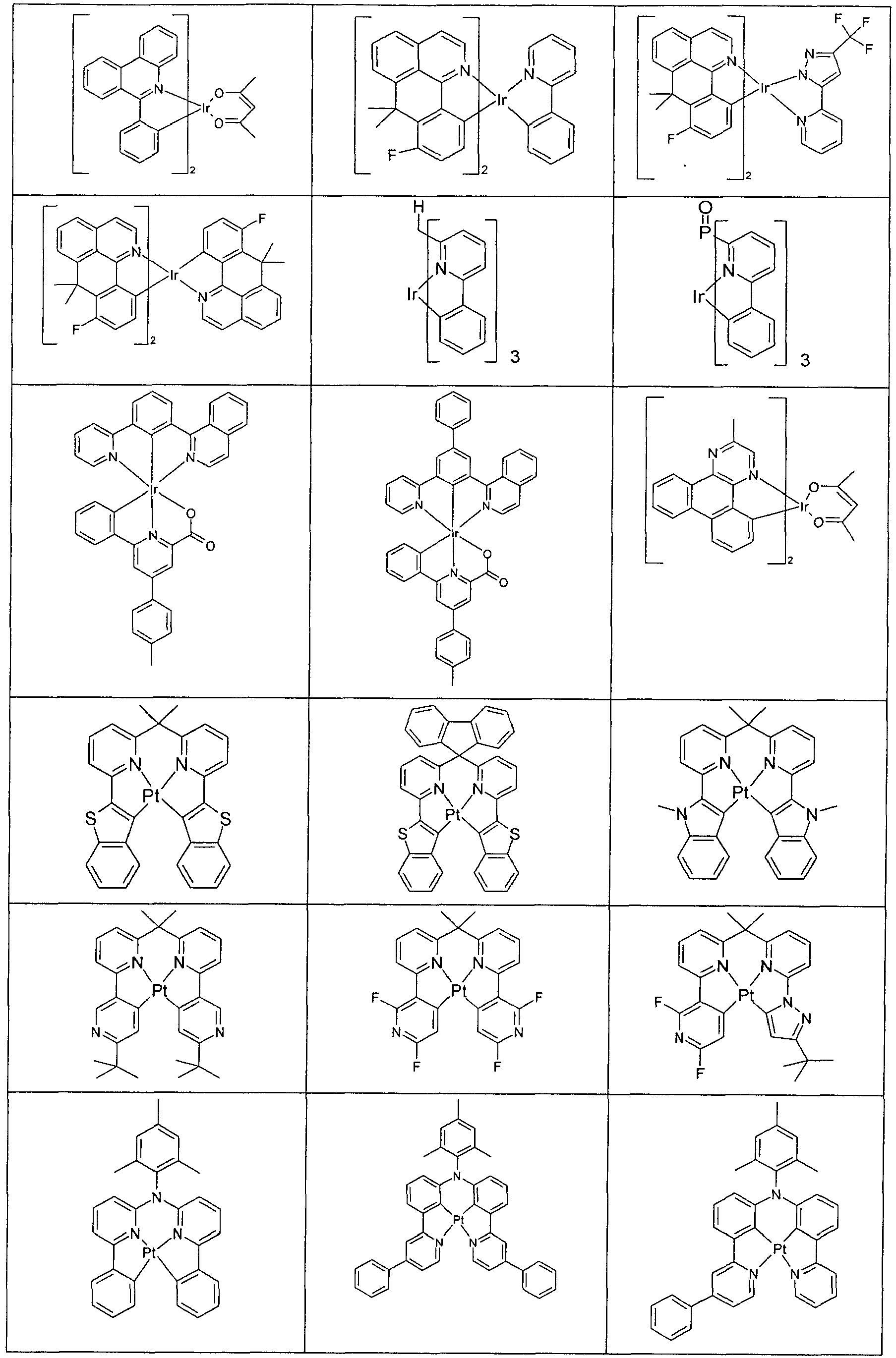

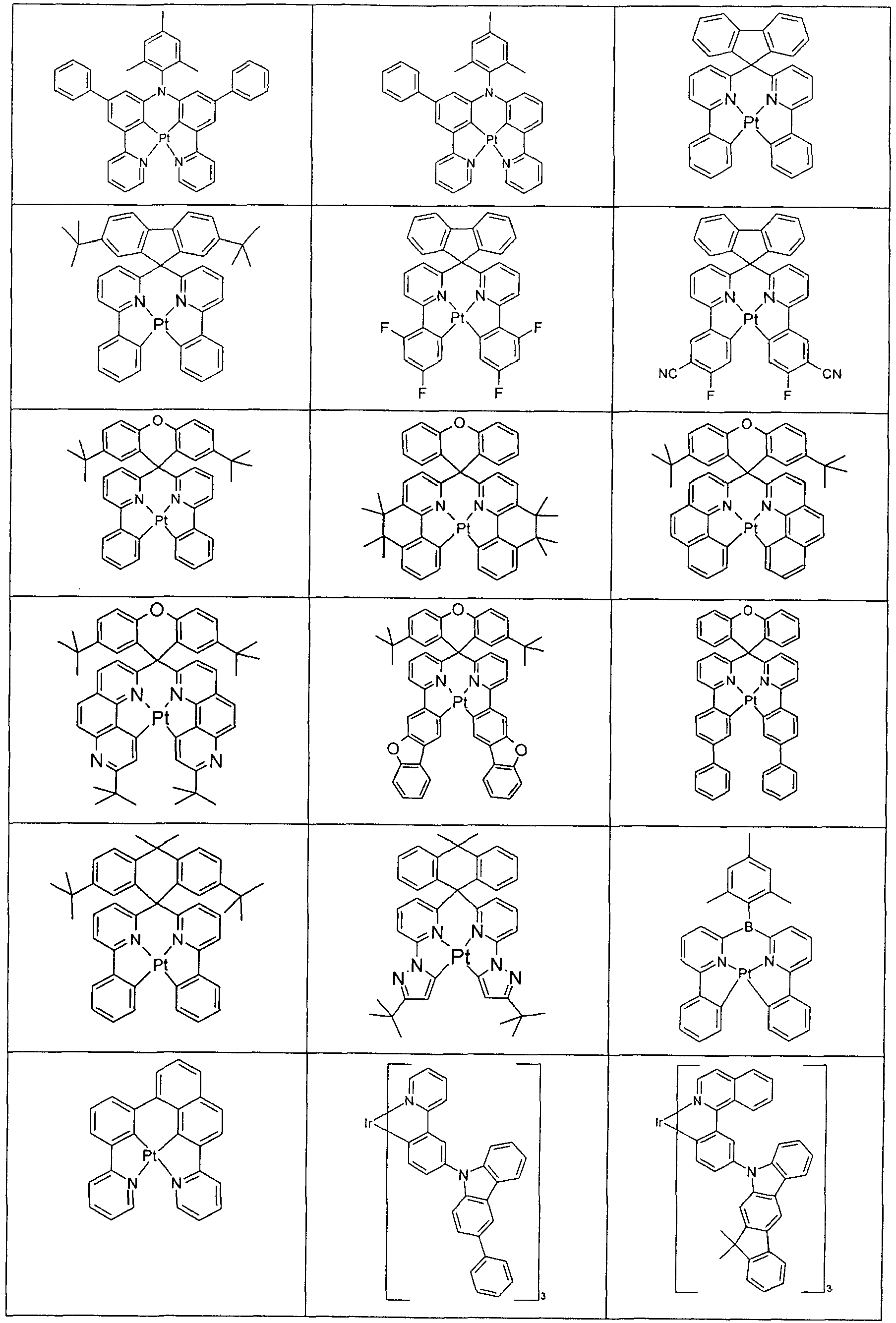

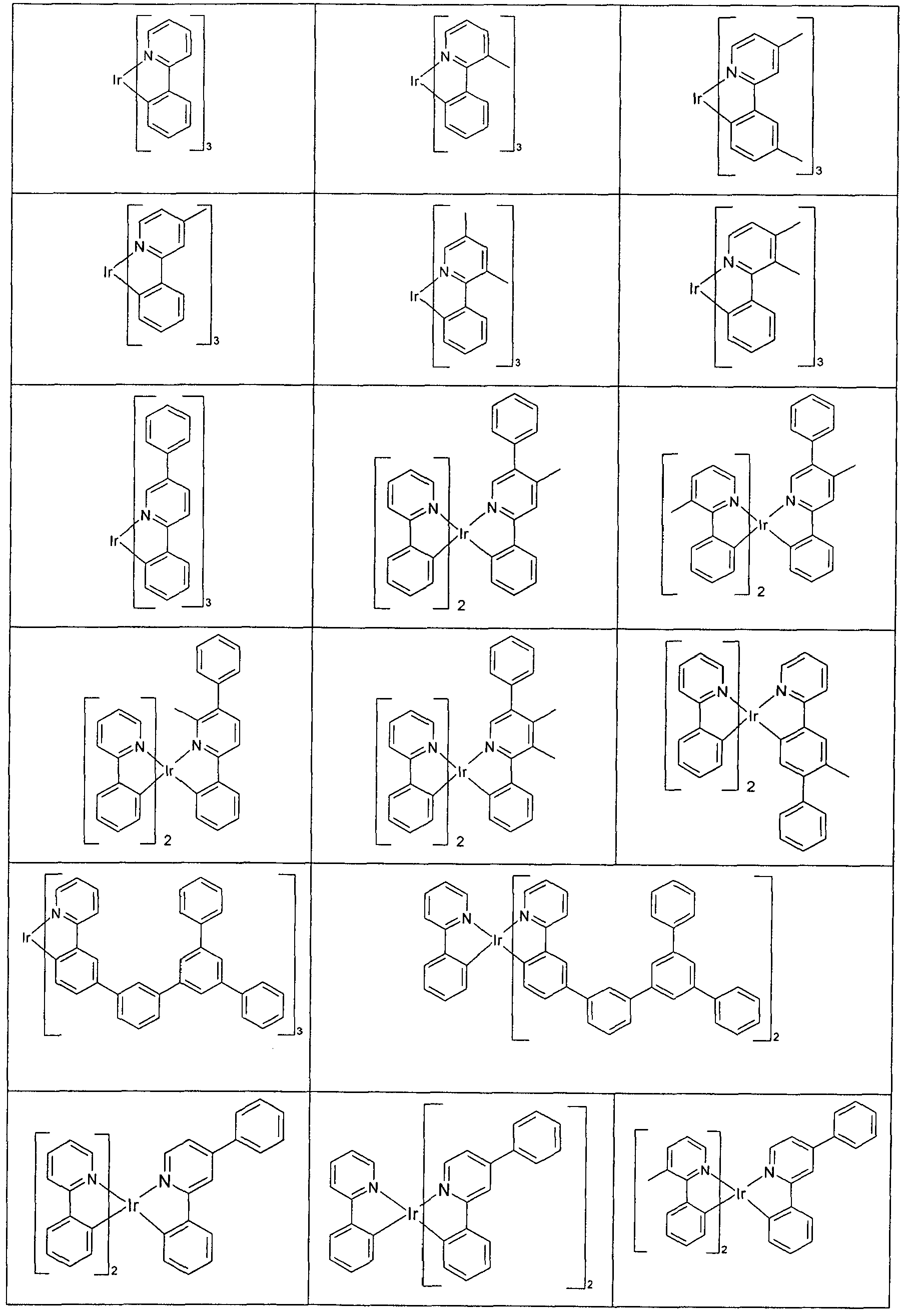

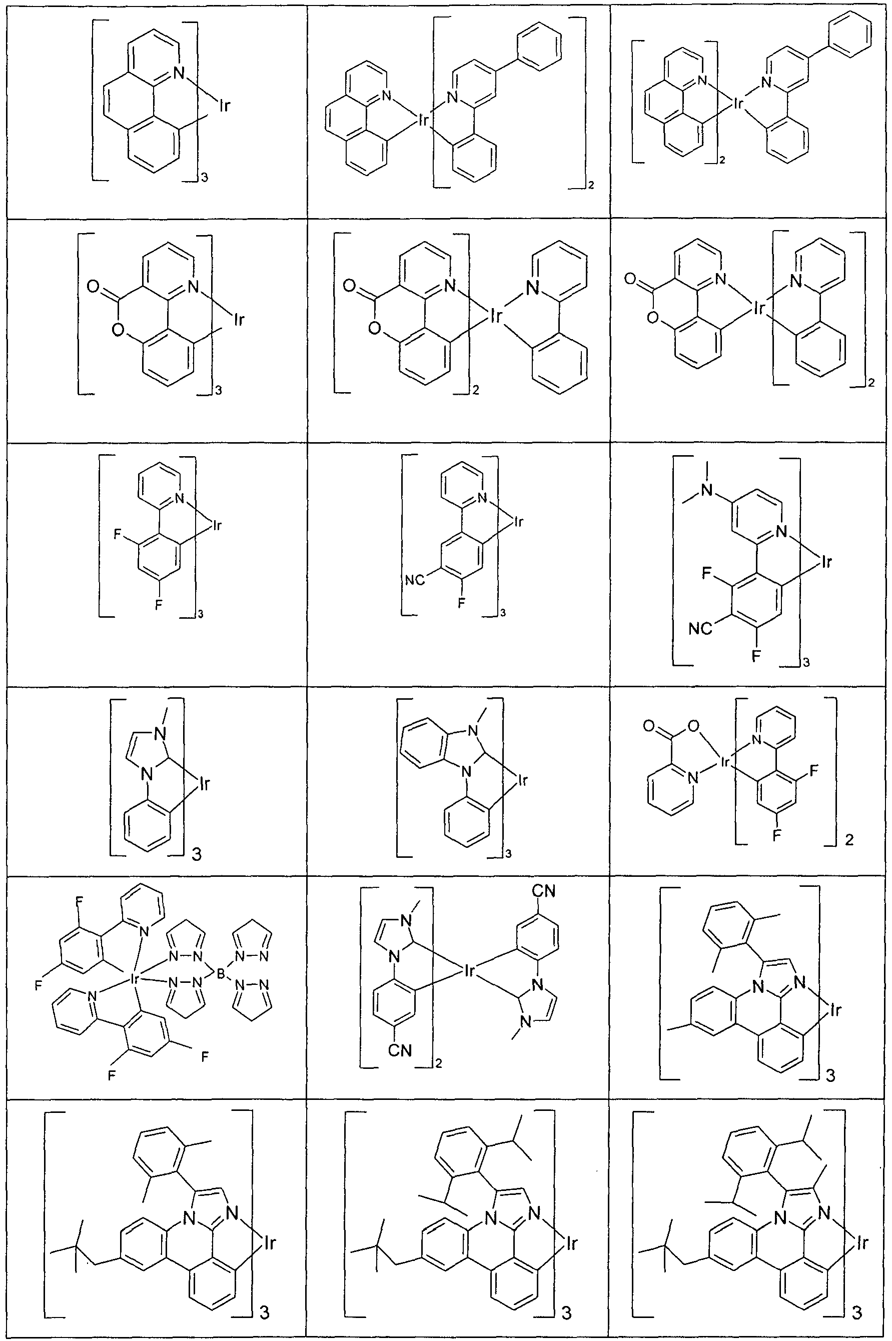

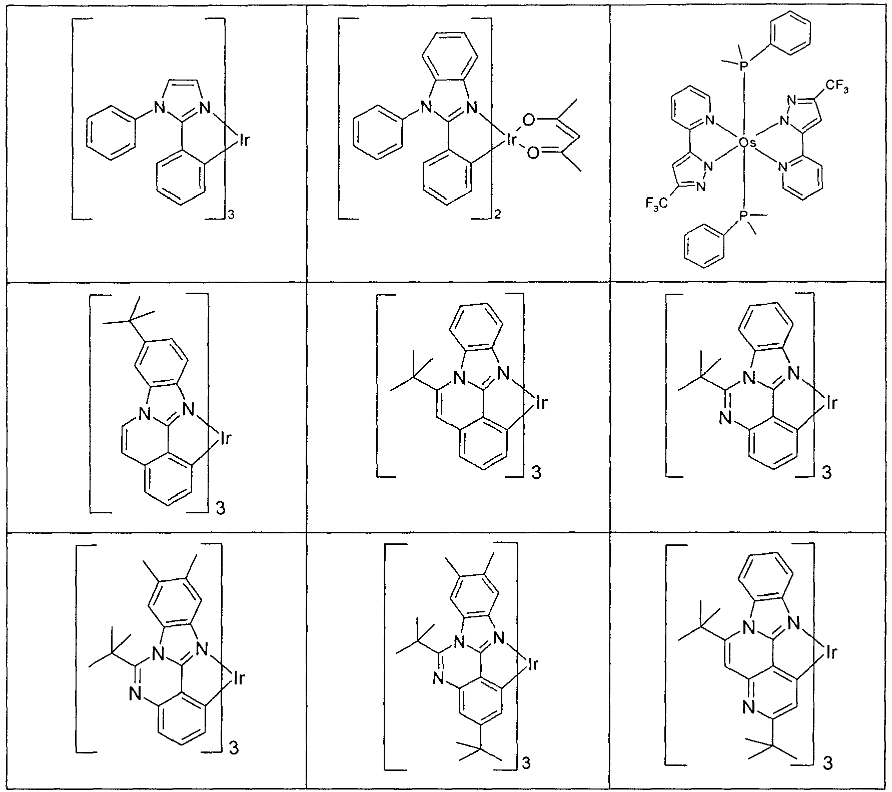

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die Verbindung gemäß Formel (1) bzw. die oben ausgeführten bevorzugten Ausführungsformen als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die Verbindung gemäß Formel (1) bzw. die oben ausgeführten bevorzugten Ausführungsformen in einer Lochblockierschicht eingesetzt. Unter einer Lochblockierschicht wird eine Schicht verstanden, die auf Kathodenseite direkt an eine emittierende Schicht angrenzt.

Es ist weiterhin möglich, die Verbindung gemäß Formel (1) bzw. die oben ausgeführten bevorzugten Ausführungsformen sowohl in einer Lochblockierschicht bzw. Elektronentransportschicht als auch als Matrix in einer emittierenden Schicht zu verwenden.

In nochmals einer weiteren Ausführungsform der Erfindung wird die Verbindung gemäß Formel (1) bzw. die oben ausgeführten bevorzugten Ausführungsformen in einer Lochtransportschicht bzw. in einer Elektronenblockierschicht bzw. Exzitonenblockierschicht eingesetzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen, eingesetzt als Matrixmaterial für fluoreszierende oder phosphoreszierende Emitter, führen zu hohen Effizienzen sowie zu langen Lebensdauern. Dies gilt insbesondere, wenn die Verbindungen als Matrixmaterial für einen rot oder grün phosphoreszierenden Emitter eingesetzt werden.
2. Die erfindungsgemäßen Verbindungen weisen eine hohe thermische Stabilität auf.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.
4. Auch bei Verwendung als Elektronentransportmaterial führen die erfindungsgemäßen Verbindungen zu sehr guten Eigenschaften in Bezug auf die Effizienz, die Lebensdauer und die Betriebsspannung von organischen Elektrolumineszenzvorrichtungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden. Die Synthese von Phenylacridon und 10-[1,1'-Diphenyl]-4-yl-acridinon sowie weiterer Derivate kann gemäß der Literatur (Chemical Communications, 48(86), 10678-10680; 2012) erfolgen und ist aus der Literatur bekannt. Die Angaben in eckigen Klammern sind die CAS-Nummern der literaturbekannten Verbindungen.

### Synthese von Edukten

### a) Synthese von (2-Bromphenyl)-phenyl-carbaminsäure tert-butyl ester

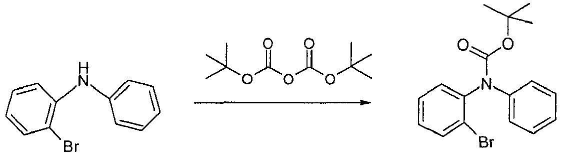

33 g (145 mmol) Di-tert-butyl-dicarbonat werden in 600 ml Toluol vorgelegt. Zu dieser Lösung werden 27 g (110 mmol) (2-Bromphenyl)-phenyl-amin und 1.3 g (11 mmol) 4-Dimethylamino-pyridin zugefügt und 40 h unter Rückfluss gekocht. Anschließend wird die Reaktion mit 200 ml Wasser versetzt, die organische Phase abgetrennt und das Lösungsmittel im Vakuum entfernt. Der Rückstand wird in der Wärme aus Hexan umkristallisiert. Ausbeute: 36 g (103 mmol), 95 %.

### b) Synthese von (2-Brom-thiophen-3-yl)-phenyl-amin

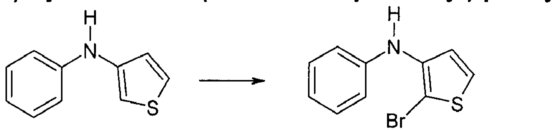

10 g (57 mmol) Phenyl-thiophen-3-yl-amin (CAS: 227805-72-3) werden in 200 mL CH₂Cl₂ vorgelegt. Anschließend tropft man unter Lichtausschluss bei -15 °C eine Lösung aus 10.1 g (57 mmol) NBS in 200 ml Acetonitril zu, lässt auf Raumtemperatur kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 11.6 g (45.6 mmol), 80 % d. Th., Reinheit nach ¹H-NMR ca. 97 %.

### Beispiel 1: Spirosynthese

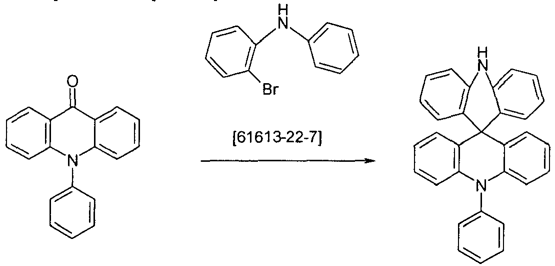

42.8 g (172 mmol) 2-Bromdiphenylamin werden in 200 ml absolutem THF, vorgelegt, auf -70 °C gekühlt und mit 150 ml (345 mmol) n-Butyllithium versetzt. Anschließend lässt man auf -10 °C kommen und rührt eine weitere Stunde bei dieser Temperatur. Dann werden langsam 30 g (86 mmol) 10-Phenyl-10H-acridin-9-on, gelöst in 500 ml THF, zugegeben und 24 h bei Raumtemperatur gerührt. Man gibt 100 ml Ammoniumchloridlösung zu, rührt kurz nach, trennt die organische Phase ab und entfernt das Lösungsmittel im Vakuum. Der Rückstand wird in der Wärme bei 40 °C in 800 ml Eisessig suspendiert, die Suspension wird mit 75 ml konz. Salzsäure versetzt und anschließend 8 h bei Raumtemperatur nachgerührt. Nach Erkalten saugt man vom ausgefallenen Feststoff ab, wäscht diesen einmal mit 100 ml Eisessig, dreimal mit je 100 ml Ethanol und kristallisiert abschließend aus Hexan um. Ausbeute: 29 g (68 mmol), 63 %; Reinheit ca. 98 % n. ¹H-NMR.

Analog können folgende Verbindungen erhalten werden:

| **Bsp.** | **Edukt 1** | **Edukt 2** | **Produkt** | **Ausbeute** |
|---|---|---|---|---|
| 1a | 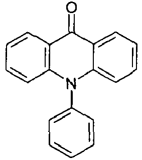 | 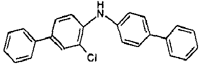 | 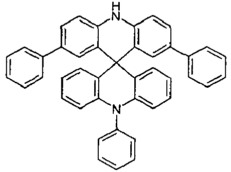 | 65% |
| | | [661572-31-8] | | |
| 1b | 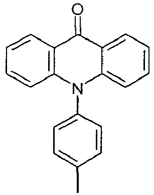 | 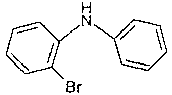 | 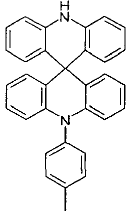 | 69 % |
| | [102023-92-7] | [61613-22-7] | | |
| 1c | 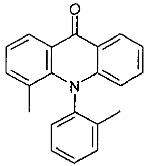 | 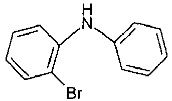 | 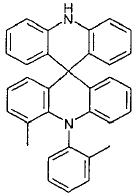 | 71% |
| | [1195562-29-8] | [61613-22-7] | | |
| 1d | 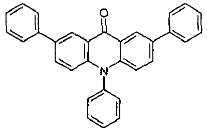 | 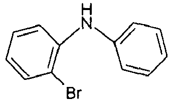 | 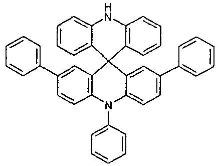 | 67% |
| | [1416006-18-4] | [61613-22-7] | | |
| 1e | 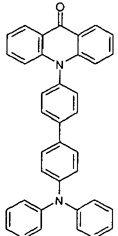 | 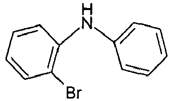 | 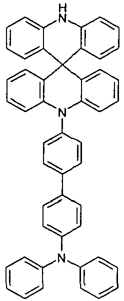 | 59% |
| | [1188546-10-2] | [61613-22-7] | | |
| 1f | 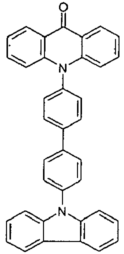 | 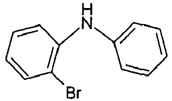 | 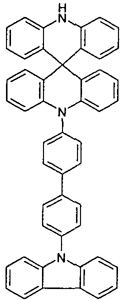 | 62% |
| | [1188546-11-2] | [61613-22-7] | | |
| 1g | 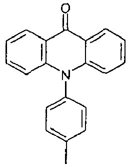 | 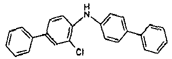 | 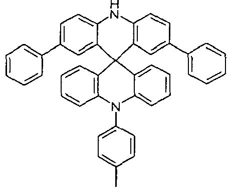 | 65% |
| | [102023-92-7] | [861572-31-8] | | |
| 1h | 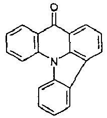 | 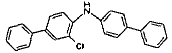 | 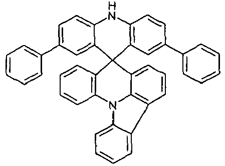 | 69% |
| | [32081-26-8] | [861572-31-8] | | |
| 1j | 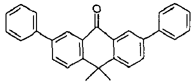 | 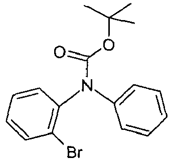 | 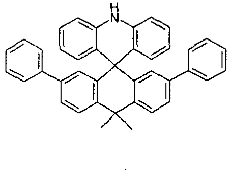 | 73% |
| | [1346009-95-7] | | | |
| 1i | 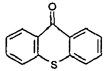 | 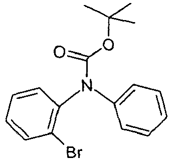 | 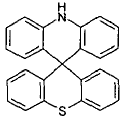 | 79% |
| | [1380298-27-0] | | | |
| 1k | 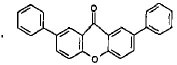 | 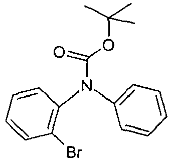 | 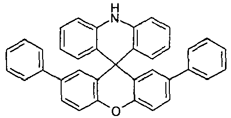 | 70% |
| | [854661-66-8] | | | |
| 1l | 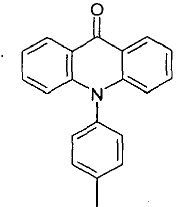 | 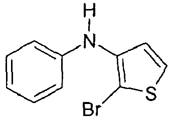 | 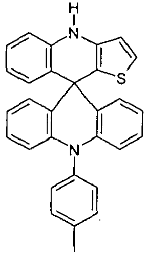 | 68% |
| | [102023-92-7] | | | |
| 1m | 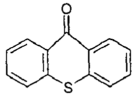 | 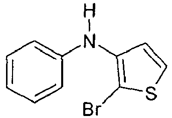 | 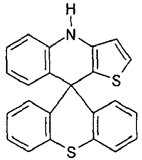 | 72% |
| | [1380298-27-0] | | | |
| 1n | 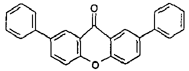 | 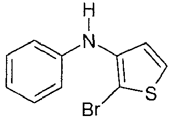 | 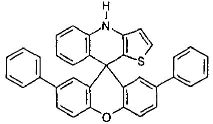 | 68% |
| | [854661-66-8] | | | |
| 1o | 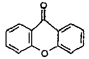 | 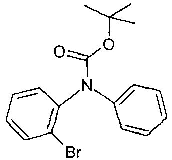 | 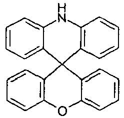 | 69% |
| | [90-47-1] | | | |
| 1p | 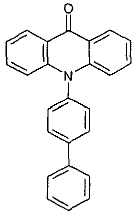 | 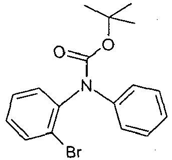 | 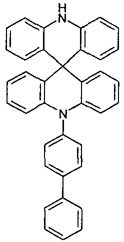 | 72% |
| | [1408293-66-2] | | | |
| 1q | 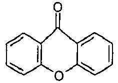 | 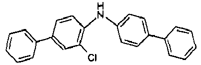 | 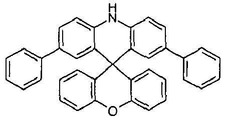 | 67% |
| | [90-47-1] | [861572-31-8] | | |

### Beispiel 2: Nukleophile Substitution (Methode 1)

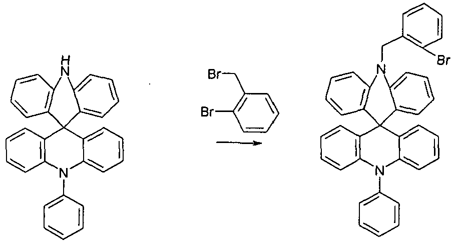

9.7 g (0.243 mol) NaH 60%ig in Mineralöl werden in 500 mL Dimethylformamid unter Schutzatmosphäre gelöst. 44 g (106 mmol) der Verbindung aus Beispiel 1 werden in 500 mL DMF gelöst und zu der Reaktionsmischung zugetropft. Nach 1 h bei Raumtemperatur wird eine Lösung von 60.6 g (242 mmol)2-Brombenzylbromid in 500 mL DMF zugetropft. Das Reaktionsgemisch wird dann 1 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol / n-Heptan umkristallisiert. Ausbeute: 55 g (93 mmol), 90%.

Analog können folgende Verbindungen erhalten werden:

| **Bsp.** | **Edukt 1** | **Edukt 2** | **Produkt** | **Ausbeute** |
|---|---|---|---|---|
| 2a | 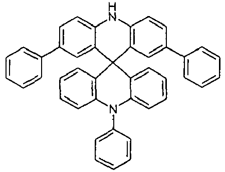 | 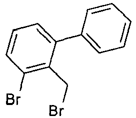 | 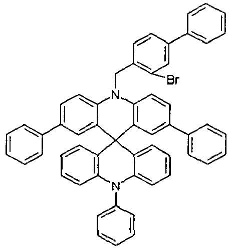 | 79% |
| | | [172976-02-2] | | |
| 2b | 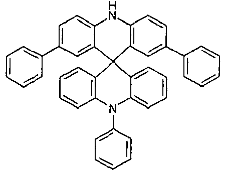 | 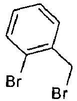 | 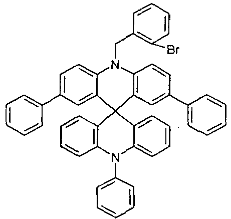 | 87 % |
| | | [3433-80-5] | | |
| 2c | 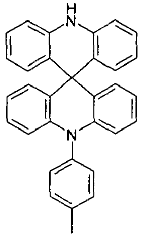 | 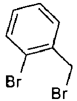 | 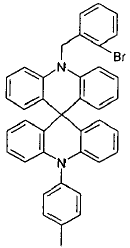 | 92 % |
| | | [3433-80-5] | | |
| 2d | 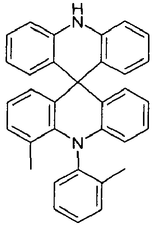 | 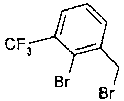 | 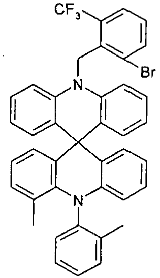 | 90 % |
| | | [1214372-35-6] | | |
| 2e | 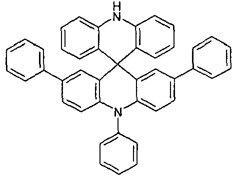 | 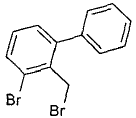 | 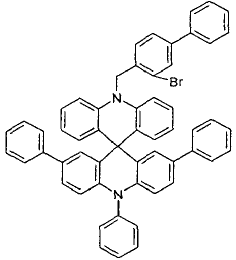 | 90 % |
| | | [172976-02-2] | | |
| 2f | 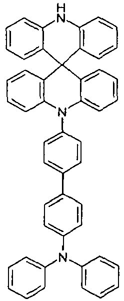 | 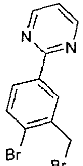 | 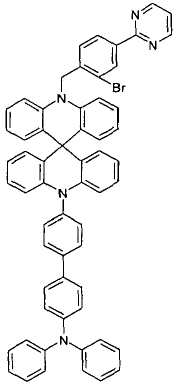 | 82 % |
| | | [202805-71-8] | | |
| 2g | 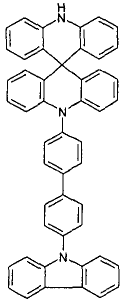 | 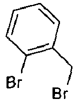 | 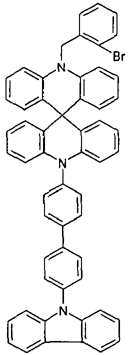 | 88% |
| | | [3433-80-5] | | |
| 2h | 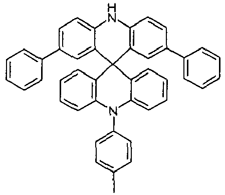 | 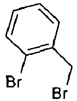 | 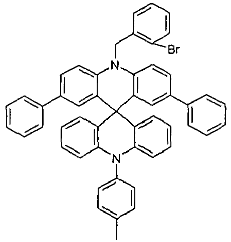 | 79% |
| | | [3433-80-5] | | |
| 2i | 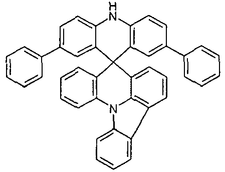 | 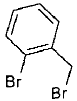 | 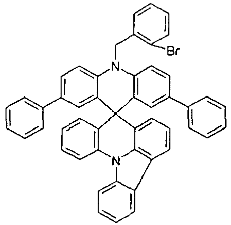 | 78% |
| | | [3433-80-5] | | |
| 2j | 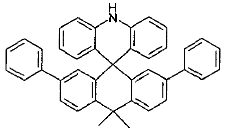 | 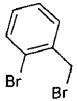 | 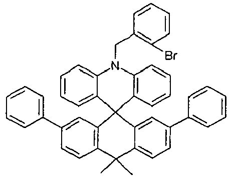 | 89% |
| | | [3433-80-5] | | |
| 2k | 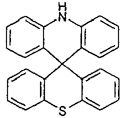 | 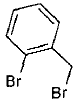 | 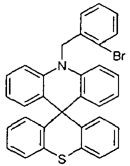 | 83% |
| | | [3433-80-5] | | |
| 2l | 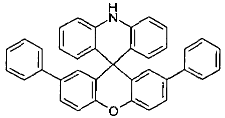 | 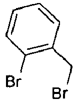 | 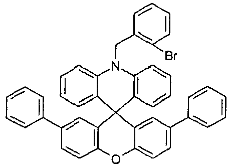 | 86% |
| | | [3433-80-5] | | |
| 2m | 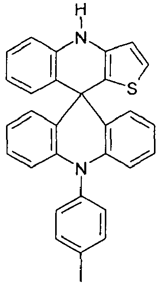 | 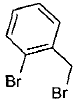 | 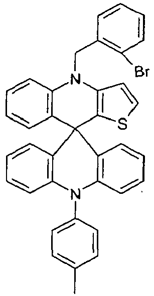 | 88% |
| | | [3433-80-5] | | |
| 2n | 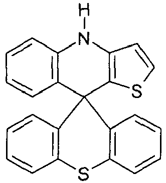 | 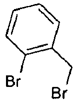 | 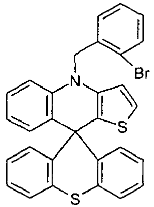 | 87% |
| | | [3433-80-5] | | |
| 2o | 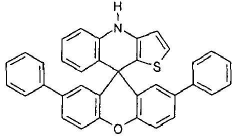 | 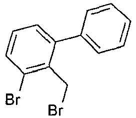 | 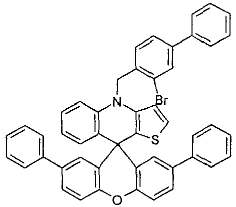 | 84% |
| | | [172976-02-2] | | |
| 2p | 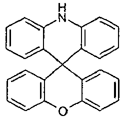 | 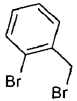 | 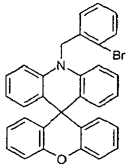 | 80% |
| | | [3433-80-5] | | |
| 2q | 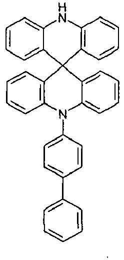 | 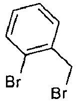 | 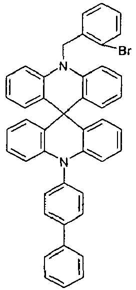 | 83% |
| | | [3433-80-5] | | |
| 2r | 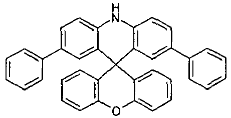 | 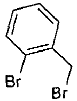 | 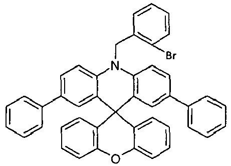 | 82% |
| | | [3433-80-5] | | |

### Beispiel 3: Cyclisierung

93 g (158 mmol) der Verbindung aus Beispiel 2 werden in 500 mL Dimethylformamid unter Schutzatmosphäre gelöst. Zur diese Lösung werden 17.3 g (0.075 mol) Benzyltrimethylammoniumbromid und 31.28 g (0.226 mol) Kaliumcarbonat zugegeben. Anschließend werden unter Schutzgas 5.08 g (0.022 mol) Pd(OAc)₂ zugegeben, und das Gemisch wird bei 120 °C für 9 h gerührt. Nach dieser Zeit wird das Reaktionsgemisch auf Raumtemperatur gekühlt und mit Dichlormethan extrahiert. Die vereinigten organischen Phasen werden über Na₂SO₄ getrocknet und eingeengt. Der Rückstand wird aus n-Heptan umkristallisiert. Ausbeute: 64 g (126 mmol), 80%.

Analog können folgende Verbindungen erhalten werden:

| **Bsp.** | **Edukt** | **Produkt** | **Ausbeute** |
|---|---|---|---|
| 3a | 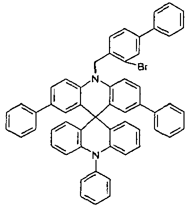 | 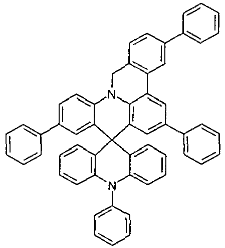 | 80% |
| 3b | 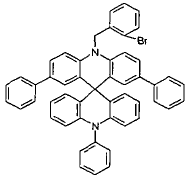 | 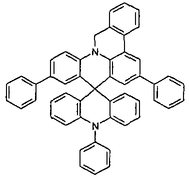 | 84% |
| 3c | 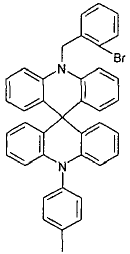 | 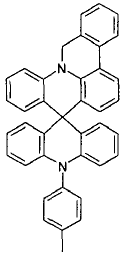 | 85 % |
| 3d | 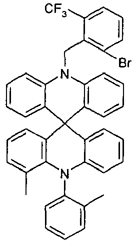 | 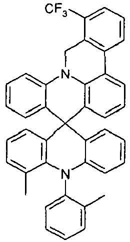 | 81 % |
| 3e | 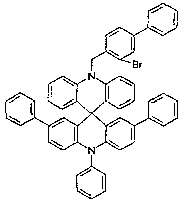 | 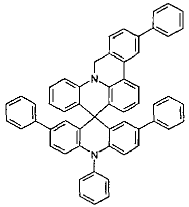 | 88% |
| 3f | 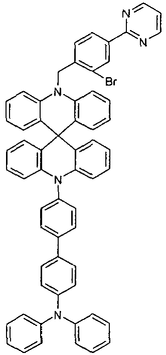 | 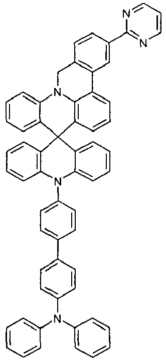 | 79% |
| 3g | 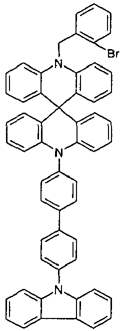 | 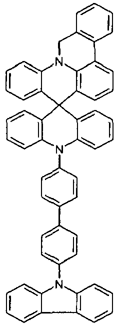 | 78 % |
| 3h | 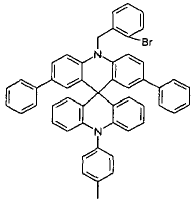 | 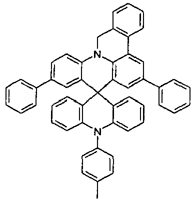 | 76% |
| 3i | 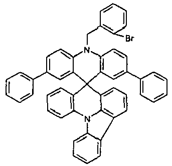 | 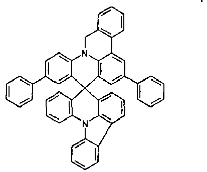 | 81% |
| 3j | 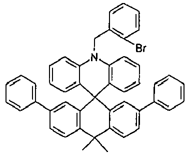 | 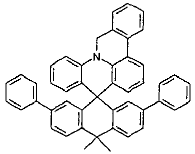 | 69% |
| 3k | 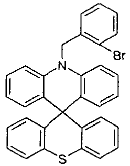 | 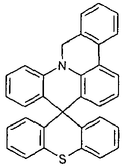 | 60% |
| 3l | 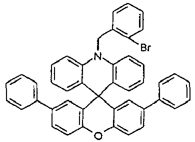 | 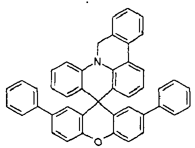 | 55% |
| 3m | 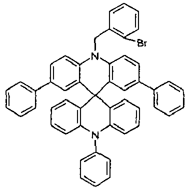 | 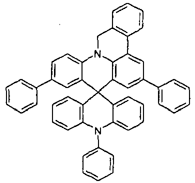 | 55% |
| 3n | 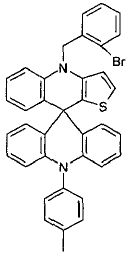 | 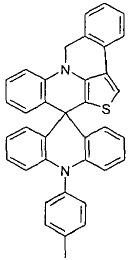 | 54% |
| 3o | 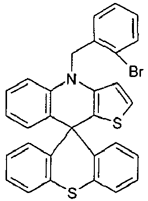 | 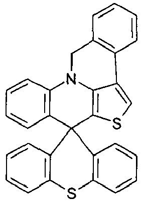 | 56% |
| 3p | 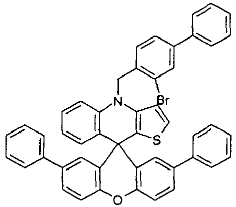 | 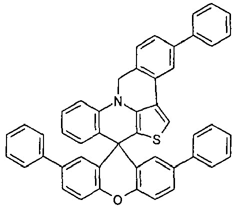 | 53% |
| 3q | 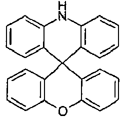 | 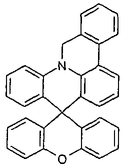 | 55% |
| 3r | 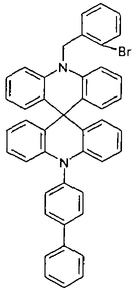 | 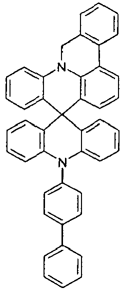 | 59% |
| 3s | 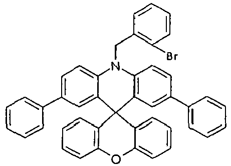 | 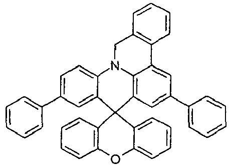 | 61% |

### Beispiel 4: Oxidation

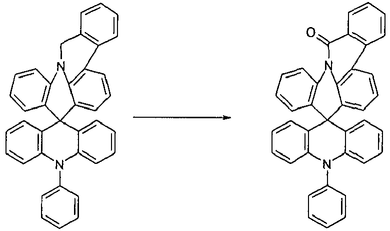

19.9 g (39 mmol) der Verbindung aus Beispiel 3 werden in 300 mL Dichlormethan gelöst. Zur diese Lösung werden 17.3 g (0.075 mol) Benzyltrimethylammoniumbromid und 62.13 g (0.393 mol) Kaliumpermanganat portionsweise zugegeben und zwei Tage bei Raumtemperatur gerührt. Nach dieser Zeit wird das restliche Kaliumpermanganat abfiltiert, die Lösung eingeengt und chromatographisch gereinigt (Laufmittel: Heptan/Dichloromethan, 5:1). Der Rückstand wird aus Toluol und aus Dichlormethan umkristallisiert und abschließend im Hochvakuum sublimiert. Ausbeute: 17 g (132 mmol), 86%, Reinheit: 99.9%.

Analog können folgende Verbindungen erhalten werden:

| **Bsp.** | **Edukt** | **Produkt** | **Ausbeute** |
|---|---|---|---|
| 4a | 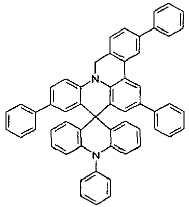 | 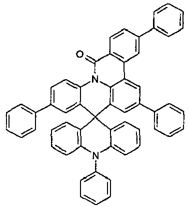 | 82% |
| 4b | 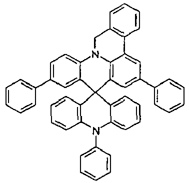 | 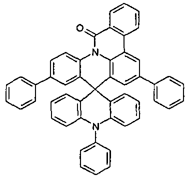 | 85 % |
| 4c | 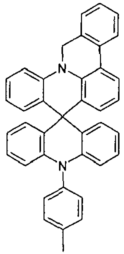 | 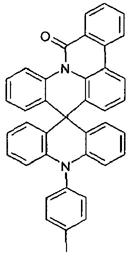 | 86 % |
| 4d | 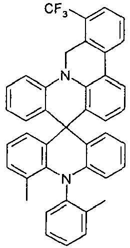 | 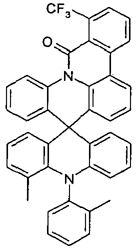 | 81 % |
| 4e | 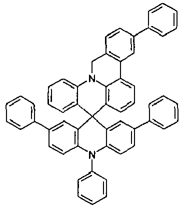 | 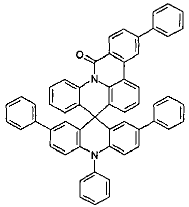 | 79 % |
| 4f | 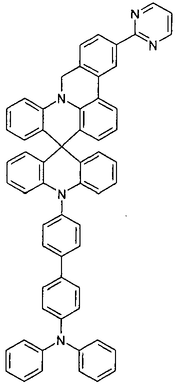 | 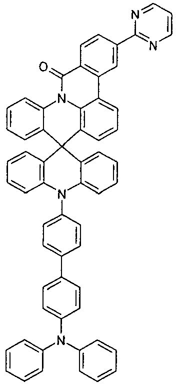 | 92% |
| 4g | 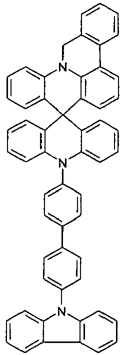 | 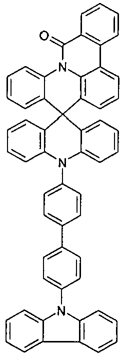 | 66 % |
| 4h | 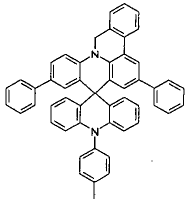 | 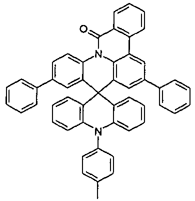 | 69% |
| 4i | 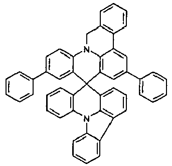 | 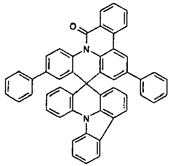 | 71% |
| 4j | 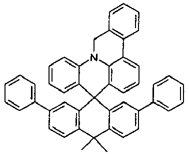 | 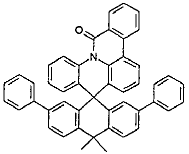 | 78% |
| 4k | 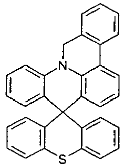 | 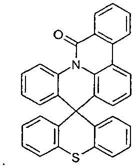 | 72% |
| 4l | 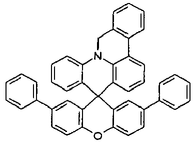 | 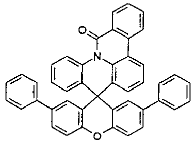 | 79% |
| 4m | 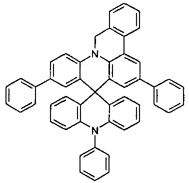 | 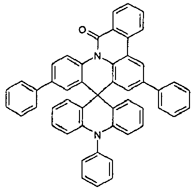 | 76% |
| 4n | 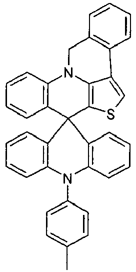 | 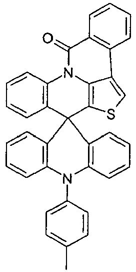 | 72% |
| 4o | 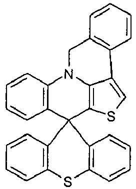 | 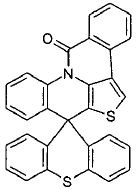 | 80% |
| 4p | 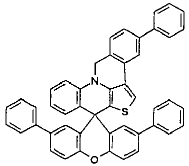 | 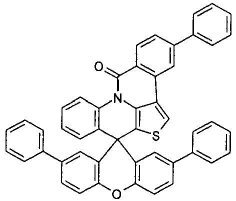 | 76% |
| 4q | 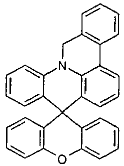 | 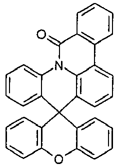 | 75% |
| 4r | 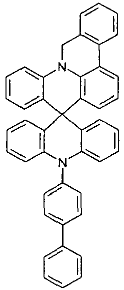 | 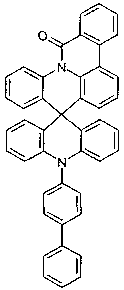 | 77% |
| 4s | 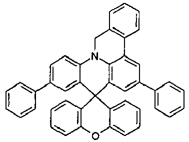 | 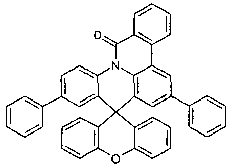 | 74% |

### Beispiel 5: Nukleophile Substitution (Methode 2)

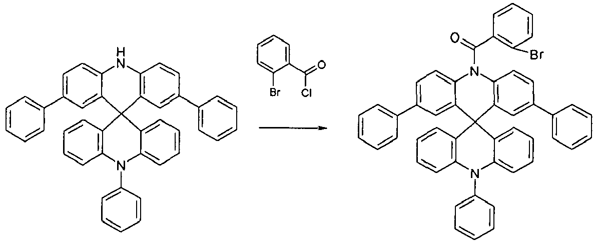

2.1 g (52.5 mmol) NaH 60%ig in Mineralöl werden in 500 mL THF unter Schutzatmosphäre gelöst. 28 g (50 mmol) der Verbindung aus Beispiel 1 und 11.5 g (52.5 mmol) 15-Crown-5, gelöst in 200 ml THF, werden zugegeben. Nach 1 h bei Raumtemperatur wird eine Lösung von 12 g (55 mmol) 2-Brom-benzoylchloride in 250 mL THF zugetropft. Das Reaktionsgemisch wird dann 18 h bei Raumtemperatur gerührt. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert und aus Toluol / n-Heptan umkristallisiert. Ausbeute: 22 g (29 mmol), 60%; Reinheit ca. 98 % n. ¹H-NMR.

Analog können folgende Verbindungen erhalten werden:

| **Bsp.** | **Edukt 1** | **Edukt 2** | **Produkt** | **Ausbeute** |
|---|---|---|---|---|
| 5a | 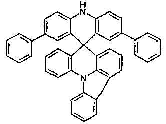 | 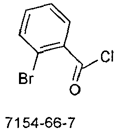 | 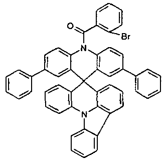 | 68% |
| 5b | 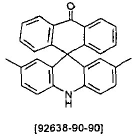 | 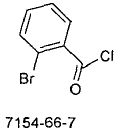 | 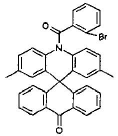 | 64% |
| 5c | 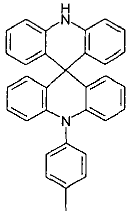 | 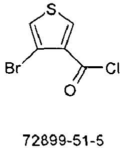 | 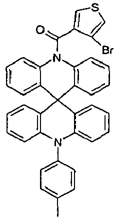 | 63% |
| 5d | 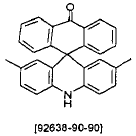 | 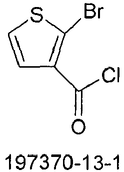 | 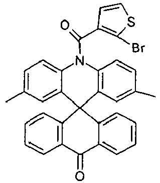 | 61% |
| 5e | 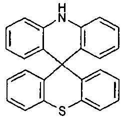 | 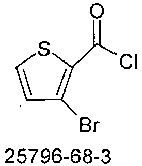 | 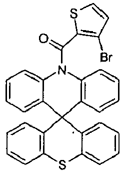 | 57% |
| 5f | 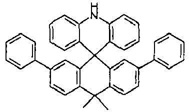 | 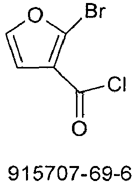 | 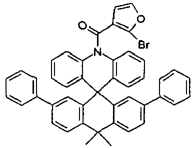 | 63% |
| 5g | 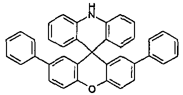 | 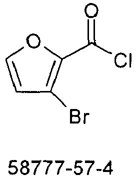 | 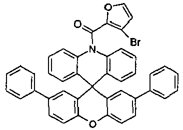 | 61% |
| 5h | 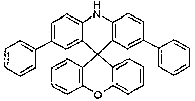 | 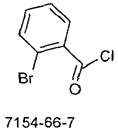 | 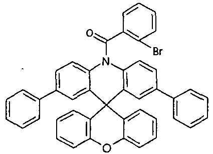 | 59% |

### Beispiel 6: Cyclisierung

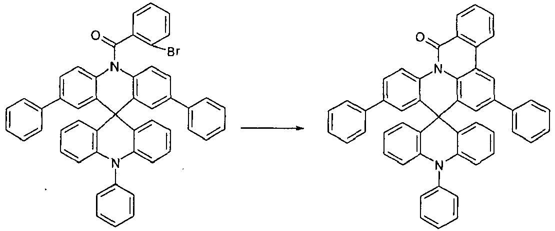

Unter Schutzgas werden 43 ml Tributylzinnhydrid (16 mmol) und 30 g (12.5 mmol) 1,1'-Azobis(cyclohexan-1-carbonitril) in 600 ml Toluol über 4 h zu einer siedenden Lösung aus 9.5 g (12.5 mmol) der Verbindung aus Beispiel 5 in 600 ml Toluol getropft. Anschließend wird 3 h unter Rückfluss gekocht. Nach dieser Zeit wird das Reaktionsgemisch auf Eis gegossen und dreimal mit Dichlormethan extrahiert. Die vereinigten organischen Phasen werden über Na₂SO₄ getrocknet und eingeengt. Der Rückstand wird aus Toluol und aus Dichlormethan / *iso*-Propanol umkristallisiert und abschließend im Hochvakuum sublimiert. Ausbeute: 5.9 g (8.7 mmol), 70 %. Reinheit 99.9%.

Analog können folgende Verbindungen erhalten werden:

| **Bsp.** | **Edukt** | **Produkt** | **Ausbeute** |
|---|---|---|---|
| 6a | 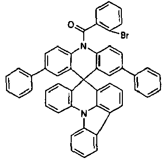 | 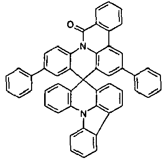 | 68% |
| 6b | 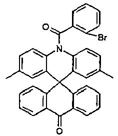 | 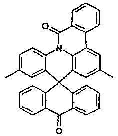 | 64% |
| 6c | 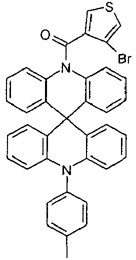 | 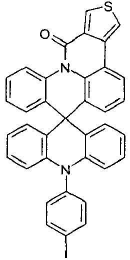 | 63% |
| 6d | 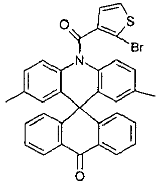 | 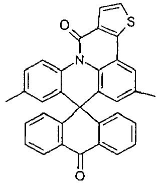 | 59% |
| 6e | 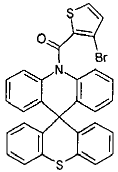 | 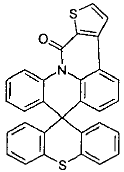 | 64% |
| 6f | 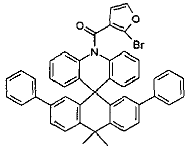 | 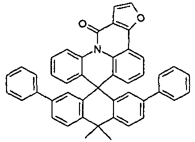 | 68% |
| 6g | 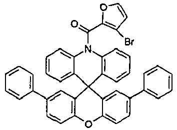 | 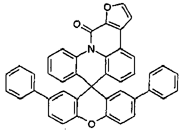 | 62% |
| 6h | 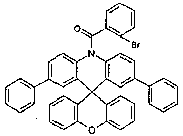 | 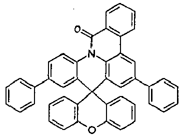 | 63% |

### Beispiel 7: Herstellung der OLEDs

In den folgenden Beispielen V1 bis E14 (siehe Tabellen 1 und 2) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylendioxythiophen)poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden. Die OLEDs haben prinzipiell folgenden Schichtaufbau: Substrat / Lochtransportschicht (HTL) / Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt. Eine Bezeichnung wie "4a" bezieht sich hierbei auf das in Beispiel 4a synthetisierte Material. Entsprechendes gilt für die anderen erfindungsgemäßen Materialien.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie 6b:IC3:TEG1 (50%:40%:10%) bedeutet hierbei, dass das Material 6b in einem Volumenanteil von 50%, IC3 in einem Anteil von 40% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiel V1-V2 sind Vergleichsbeispiele gemäß dem Stand der Technik, die Beispiele E1-E14 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt.

### Verwendung von erfindungsgemäßen Verbindungen als Matrixmaterialien in phosphoreszierenden OLEDs

Durch Austausch des 5-Rings (Stand der Technik) in der Spirogruppe des Lactams gegen einen 6-Ring (erfindungsgemäß) erhält man eine Verbesserung der Leistungseffizienz um etwa 40% bei Einsatz als Matrixmaterial (Beispiele V1, E2). Weiterhin erhält man mit erfindungsgemäßen Verbindungen eine deutlich verbesserte Betriebslebensdauer. Während bei einer OLED mit der Verbindung StdT1 als Matrixmaterial bei Betrieb mit 20 mA/cm² die anfängliche Leuchtdichte nach 90 h (Beispiel V1) bzw. 115 h (Beispiel V2) auf 80% abfällt, ist dies bei einer entsprechenden OLED mit der Verbindung 4s erst nach 160 h der Fall (Beispiel E1), d. h. man erhält eine um mehr als 40% gesteigerte Lebensdauer. Mit Verbindung 4i fällt die anfängliche Leuchtdichte nach 120 h auf 80% ab (Beispiel E2).

**Tabelle 1: Aufbau der OLEDs**

| Bsp. | HTL Dicke | IL Dicke | EBL Dicke | EML Dicke | HBL Dicke | ETL Dicke | EIL Dicke |
|---|---|---|---|---|---|---|---|
| V1 | SpA1 | HATCN | SpMA1 | StdT1:TEG1 | IC1 | ST2:LiQ | --- |
| | 70nm | 5nm | 90nm | (90%:10%) 30nm | 10nm | (50%:50%) 30nm | |
| V2 | SpA1 | HATCN | SpMA1 | StdT1:TEG1 | --- | ST2:LiQ | --- |
| | 70nm | 5nm | 90nm | (90%:10%) 30nm | | (50%:50%) 40nm | |
| E1 | SpA1 | HATCN | SpMA1 | 4s:TEG1 | --- | ST2:LiQ | --- |
| | 70nm | 5nm | 90nm | (90%:10%) 30nm | | (50%:50%) 40nm | |
| E2 | SpA1 | HATCN | SpMA1 | 4i:TEG1 | IC1 | ST2:LiQ | --- |
| | 70nm | 5nm | 90nm | (90%:10%) 30nm | 10nm | (50%:50%) 30nm | |
| E3 | SpA1 | HATCN | SpMA1 | 4j:TEG1 | IC1 | ST2:LiQ | --- |
| | 70nm | 5nm | 90nm | (90%:10%) 30nm | 10nm | (50%:50%) 30nm | |
| E4 | SpA1 | HATCN | SpMA1 | 4k:TEG1 | IC1 | ST2:LiQ | --- |
| | 70nm | 5nm | 90nm | (90%:10%) 30nm | 10nm | 50%:50%) 30nm | |
| E5 | SpA1 | HATCN | SpMA1 | 4r:IC2:TEG1 | IC1 | ST2:LiQ | --- |
| | 70nm | 5nm | 90nm | (45%:45%:10%) 30nm | 10nm | (50%:50%) 30nm | |
| E6 | SpA1 | HATCN | SpMA1 | 4q:IC2:TEG1 | IC1 | ST2:LiQ | --- |
| | 70nm | 5nm | 90nm | (45%:45%:10%) 30nm | 10nm | (50%:50%) 30nm | |
| E7 | SpA1 | HATCN | SpMA1 | 4a:IC1 :TEG1 | IC1 | ST2:LiQ | --- |
| | 70nm | 5nm | 90nm | (45%:45%:10%) 30nm | 10nm | (50%:50%) 30nm | |
| E8 | SpA1 | HATCN | SpMA1 | 4b:IC1 :TEG1 | IC1 | ST2:LiQ | --- |
| | 70nm | 5nm | 90nm | (45%:45%:10%) 30nm | 10nm | (50%:50%) 30nm | |
| E9 | SpA1 | HATCN | SpMA1 | 4e:IC1 :TEG1 | IC1 | ST2:LiQ | --- |
| | 70nm | 5nm | 90nm | (45%:45%:10%) 30nm | 10nm | (50%:50%) 30nm | |
| E10 | SpA1 | HATCN | SpMA1 | 4p:IC2:TER1 | --- | ST2:LiQ | --- |
| | 90nm | 5nm | 130nm | (30%:62%:8%) 40nm | | (50%:50%) 40nm | |
| E11 | SpA1 | HATCN | SpMA1 | 4g:IC2:TEG1 | IC1 | ST2:LiQ | --- |
| | 70nm | 5nm | 90nm | (45%:45%:10%) 30nm | 10nm | (50%:50%) 30nm | |
| E12 | SpA1 | HATCN | SpMA1 | 6b:IC3:TEG1 | ST2 | ST2:LiQ | --- |
| | 70nm | 5nm | 90nm | (50%:40%:10%) 30nm | 10nm | (50%:50%) 30nm | |
| E13 | SpA1 | HATCN | SpMA1 | IC2:TEG1 | --- | 41 | LiQ |
| | 70nm | 5nm | 90nm | (90%:10%) 30nm | | 40nm | 3nm |
| E14 | SpA1 | HATCN | SpMA1 | IC1:TEG1 | 4s | ST2:LiQ | --- |
| | 70nm | 5nm | 90nm | (90%:10%) 30nm | 10nm | (50%:50%) 30nm | |

**Tabelle 2: Daten der OLEDs**

| Bsp. | U1000 (V) | SE1000 (cd/A) | LE1000 (lm/W) | EQE 1000 | CIE x/y bei 1000 cd/m² |
|---|---|---|---|---|---|
| V1 | 4.1 | 49 | 38 | 13.7% | 0.33/0.63 |
| V2 | 3.8 | 52 | 43 | 14.0% | 0.33/0.63 |
| E1 | 3.6 | 51 | 45 | 14.3% | 0.33/0.62 |
| E2 | 3.5 | 61 | 54 | 16.6% | 0.35/0.62 |
| E3 | 3.8 | 55 | 45 | 15.5% | 0.33/0.62 |
| E4 | 4.6 | 51 | 33 | 14.3% | 0.33/0.62 |
| E5 | 3.3 | 60 | 57 | 16.1% | 0.34/0.62 |
| E6 | 3.3 | 64 | 62 | 17.3% | 0.34/0.62 |
| E7 | 3.2 | 56 | 56 | 15.8% | 0.33/0.62 |
| E8 | 3.4 | 57 | 52 | 15.9% | 0.33/0.63 |
| E9 | 3.4 | 58 | 54 | 16.2% | 0.33/0.62 |
| E10 | 4.4 | 11.0 | 7.9 | 11.9% | 0.67/0.33 |
| E11 | 3.1 | 57 | 57 | 15.8% | 0.33/0.62 |
| E12 | 4.2 | 40 | 29 | 11.1% | 0.33/0.62 |
| E13 | 4.6 | 43 | 36 | 14.9% | 0.33/0.62 |
| E14 | 4.7 | 51 | 34 | 14.3% | 0.33/0.62 |

**Tabelle 3: Strukturformeln der Materialien für die OLEDs**

| | |
|---|---|
| 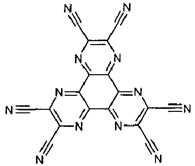 | 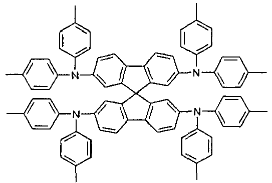 |
| HATCN | SpA1 |
| 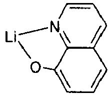 | 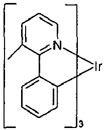 |
| LiQ | TEG1 |
| 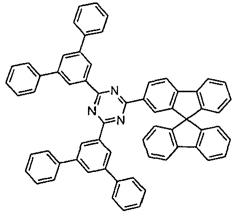 | 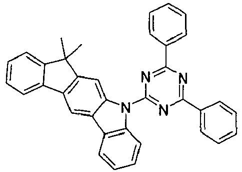 |
| ST2 | IC1 |
| 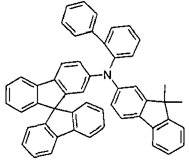 | 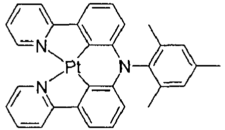 |
| SpMA1 | TER1 |
| 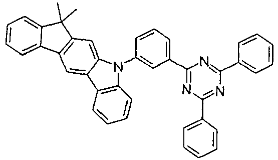 | 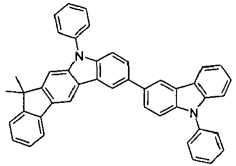 |
| IC2 | IC3 |
| 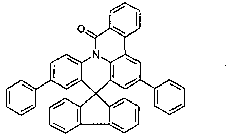 | |
| StdT1 | |

## Patentansprüche

1. Verbindung gemäß Formel (1), 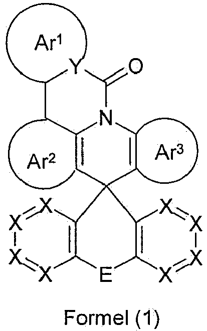 wobei für die verwendeten Symbole und Indizes gilt:
X ist bei jedem Auftreten gleich oder verschieden CR oder N;
Y ist C, wenn Ar¹ eine 6-Ring-Aryl- oder Heteroarylgruppe darstellt, bzw. ist C oder N, wenn Ar¹ eine 5-Ring-Heteroarylgruppe darstellt;
E ist NR, CR₂, O, S oder C=O;
Ar¹ ist zusammen mit der Gruppe Y und dem explizit dargestellten Kohlenstoffatom eine Gruppe der Formel (2), (3), (4), (5) oder (6), 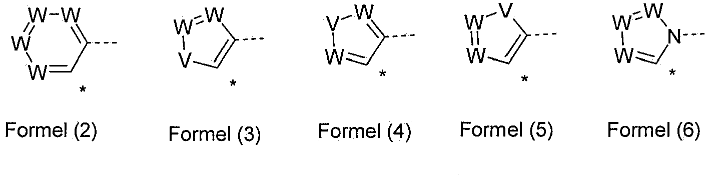 wobei die gestrichelte Bindung die Verknüpfung mit der Carbonylgruppe des Lactams andeutet, * die Position der Verknüpfung mit Ar² andeutet und weiterhin gilt:
W ist gleich oder verschieden bei jedem Auftreten CR oder N;
V ist NR, O oder S;
Ar² ist zusammen mit den explizit dargestellten Kohlenstoffatomen eine Gruppe gemäß einer der Formeln (9), (10) oder (11); 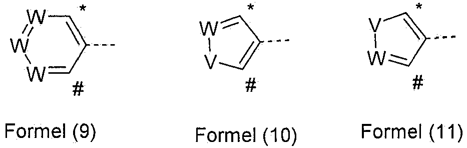 wobei die gestrichelte Bindung die Verknüpfung mit N andeutet, # die Position der Verknüpfung mit dem Spiro-Kohlenstoffatom andeutet, * die Verknüpfung mit Ar¹ andeutet und W und V die oben genannten Bedeutungen aufweisen,
Ar³ ist zusammen mit den explizit dargestellten Kohlenstoffatomen eine Gruppe gemäß einer der Formeln (12), (13), (14) oder (15), 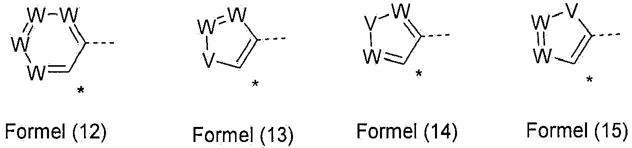 wobei die gestrichelte Bindung die Verknüpfung mit N andeutet, * die Verknüpfung mit dem Spiro-Kohlenstoffatom andeutet und W und V die oben genannten Bedeutungen aufweisen;
R ist bei jedem Auftreten gleich oder verschieden ausgewählt aus der Gruppe bestehend aus H, D, F, Cl, Br, CN, N(Ar⁴)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R¹ substituiert sein kann, einer Aryloxy- oder Heteroaryloxygruppe mit 5 bis 30 aromatischen Ringatomen, die mit einem oder mehreren Resten R¹ substituiert sein kann, wobei optional zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann;
R¹ ist bei jedem Auftreten gleich oder verschieden ausgewählt aus der Gruppe bestehend aus H, D, F, Cl, Br, CN, einer geradkettigen Alkyl- oder Alkoxygruppe mit 1 bis 40 C-Atomen oder einer verzweigten oder cyclischen Alkyl- oder Alkoxygruppe mit 3 bis 40 C-Atomen oder einer Alkenylgruppe mit 2 bis 40 C-Atomen, die jeweils mit einem oder mehreren Resten R² substituiert sein kann, wobei ein oder mehrere H-Atome durch D oder F ersetzt sein können, einem aromatischen oder heteroaromatischen Ringsystem mit 5 bis 60 aromatischen Ringatomen, das jeweils mit einem oder mehreren Resten R² substituiert sein kann;
Ar⁴ ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar⁴, welche an dasselbe N-Atom binden, auch durch eine Einfachbindung oder eine Brücke, ausgewählt aus N(R²), C(R²)₂ oder O, miteinander verbrückt sein;
R² ist ausgewählt aus der Gruppe bestehend aus H, D, F, CN oder einem aliphatischem Kohlenwasserstoffrest mit 1 bis 20 C-Atomen.

2. Verbindung nach Anspruch 1, **dadurch gekennzeichnet, dass** maximal eine Gruppe X pro Cyclus für N steht.

3. Verbindung nach Anspruch 1 oder 2, ausgewählt aus den Verbindungen der Formeln (16) bis (25), 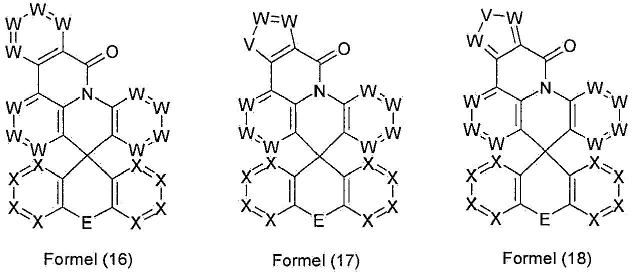 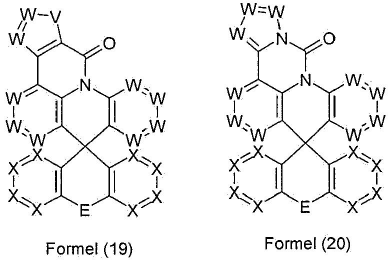 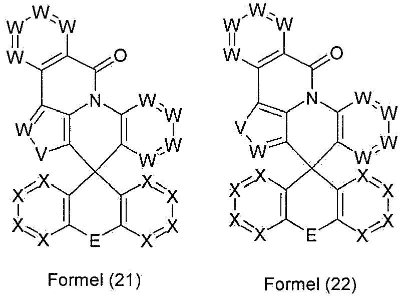 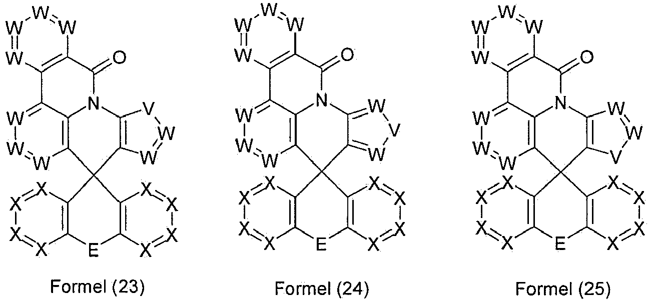 wobei die verwendeten Symbole die in Anspruch 1 genannten Bedeutungen aufweisen.

4. Verbindung nach einem oder mehreren der Ansprüche 1 bis 3, ausgewählt aus den Verbindungen der Formeln (16a) bis (25a), 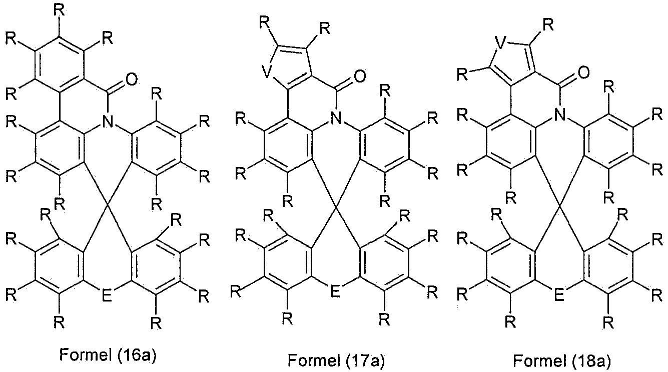 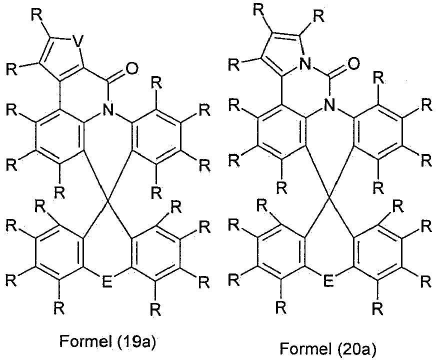 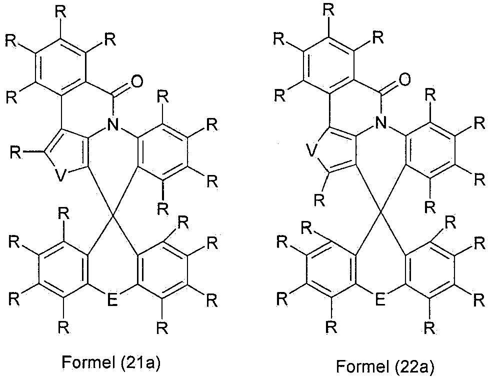 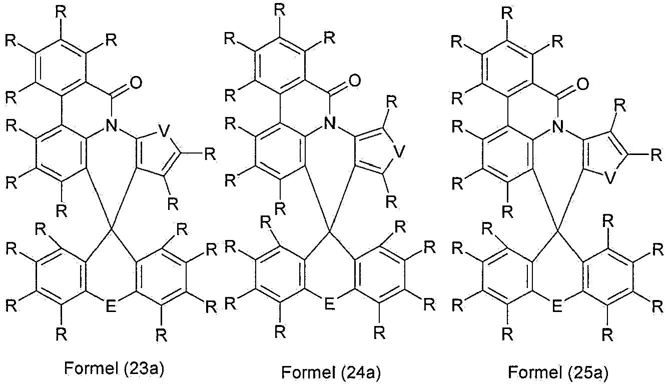 wobei die verwendeten Symbole die in Anspruch 1 genannten Bedeutungen aufweisen.

5. Verbindung nach einem oder mehreren der Ansprüche 1 bis 4, ausgewählt aus den Verbindungen gemäß den Formeln (16b) bis (25b), 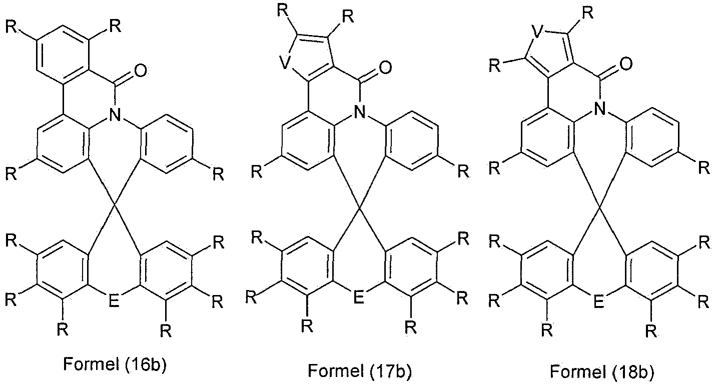 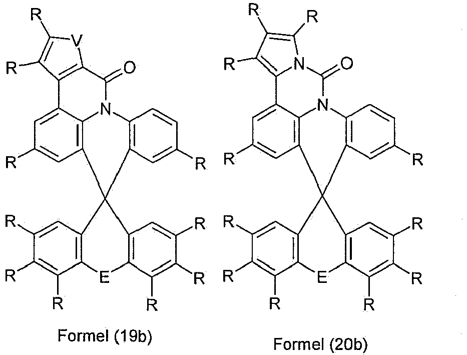 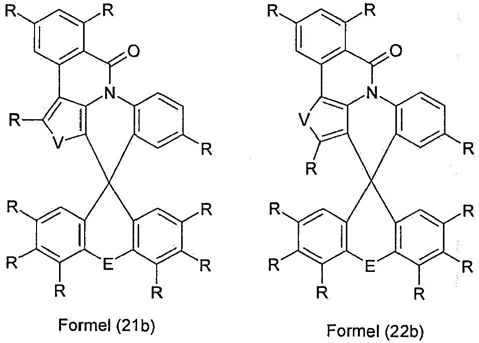 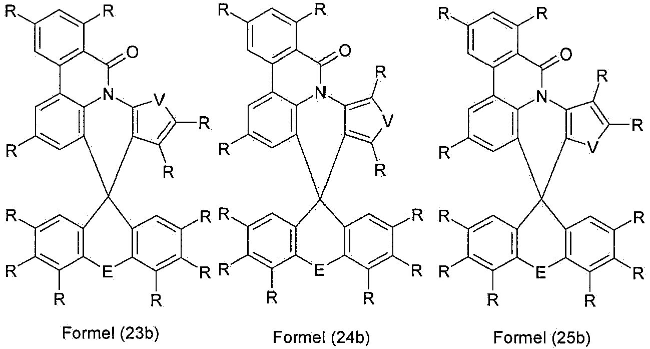 wobei die verwendeten Symbole die in Anspruch 1 genannten Bedeutungen aufweisen.

6. Verbindung nach einem oder mehreren der Ansprüche 1 bis 5, **dadurch gekennzeichnet, dass** E für NR steht und R ausgewählt ist aus aromatischen und heteroaromatischen Ringsystemen mit 5 bis 40 aromatischen Ringatomen, welche mit einem oder mehreren Resten R¹ substituiert sein können, insbesondere Phenyl, ortho-, meta- oder para-Biphenyl, ortho-, meta-, para- oder verzweigtes Terphenyl, ortho-, meta-, para- oder verzweigtes Quaterphenyl, 1-, 2-, 3- oder 4-Fluorenyl, 1-, 2-, 3- oder 4-Spirobifluorenyl, Triazin, 1-, 2-, 3- oder 4-Dibenzofuranyl, 1-, 2-, 3- oder 4-Dibenzothienyl oder 1-, 2-, 3- oder 4-Carbazolyl, die jeweils durch einen oder mehrere Reste R¹ substituiert sein können.

7. Formulierung, insbesondere eine Lösung oder Dispersion, enthaltend mindestens eine Verbindung nach einem oder mehreren der Ansprüche 1 bis 6 und mindestens eine weitere Verbindung, insbesondere ein Lösemittel.

8. Verfahren zur Herstellung einer Verbindung nach einem oder mehreren der Ansprüche 1 bis 6, umfassend die Reaktionsschritte:
a) Herstellung einer Spiroverbindung ausgehend von einem Acridin-, Xanthon-, Thioxanthenon- oder Anthracen-9-on-derivat; und
b) Substitution des Stickstoffs gefolgt von Cyclisierung zum Lactam.

9. Verwendung einer Verbindung nach einem oder mehreren der Ansprüche 1 bis 6 in einer elektronischen Vorrichtung, insbesondere in organischen Elektrolumineszenzvorrichtung.

10. Elektronische Vorrichtung, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoffsensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices" enthaltend eine oder mehrere Verbindungen nach einem oder mehreren der Ansprüche 1 bis 6.

11. Elektronische Vorrichtung nach Anspruch 10, wobei es sich um eine organische Elektrolumineszenzvorrichtung handelt, **dadurch gekennzeichnet, dass** die Verbindung nach einem oder mehreren der Ansprüche 1 bis 6 als Matrixmaterial für fluoreszierende oder phosphoreszierende Emitter und/oder in einer Lochblockierschicht und/oder in einer Elektronentransportschicht und/oder in einer elektronenblockierenden bzw. exzitonenblockierenden Schicht und/oder in einer Lochtransportschicht eingesetzt wird.

## Claims

1. Compound of formula (1) 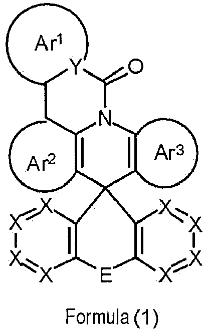 where the symbols and indices used are as follows:
X is the same or different at each instance and is CR or N;
Y is C when Ar¹ is a 6-membered aryl or heteroaryl group, or is C or N when Ar¹ is a 5-membered heteroaryl group;
E is NR, CR₂, O, S or C=O;
Ar¹ together with the Y group and the carbon atom shown explicitly is a group of the formula (2), (3), (4), (5) or (6) 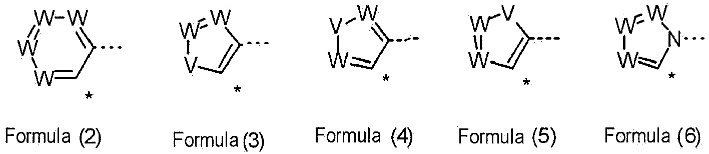 where the dotted bond indicates the linkage to the carbonyl group of the lactam, * indicates the position of the linkage to Ar² and in addition:
W is the same or different at each instance and is CR or N;
V is NR, O or S;
Ar² together with the carbon atoms shown explicitly is a group of one of the formulae (9), (10) and (11) 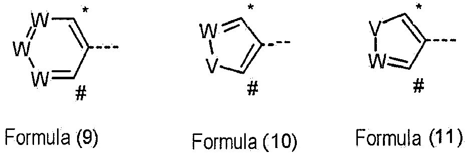 where the dotted bond indicates the linkage to N, # indicates the position of the linkage to the spiro carbon atom, * indicates the linkage to Ar¹ and W and V are each as defined above,
Ar³ together with the carbon atoms shown explicitly is a group of one of the formulae (12), (13), (14) and (15) 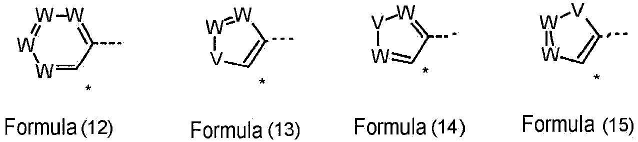 where the dotted bond indicates the linkage to N, * indicates the linkage to the spiro carbon atom and W and V are each as defined above;
R is the same or different at each instance and is selected from the group consisting of H, D, F, Cl, Br, CN, N(Ar⁴)₂, a straight-chain alkyl or alkoxy group having 1 to 10 carbon atoms or a branched or cyclic alkyl or alkoxy group having 3 to 10 carbon atoms or an alkenyl group having 2 to 10 carbon atoms, each of which may be substituted by one or more R¹ radicals, where one or more nonadjacent CH₂ groups may be replaced by O and where one or more hydrogen atoms may be replaced by D or F, an aromatic or heteroaromatic ring system which has 5 to 30 aromatic ring atoms and may be substituted in each case by one or more R¹ radicals, an aryloxy or heteroaryloxy group which has 5 to 30 aromatic ring atoms and may be substituted by one or more R¹ radicals, where it is optionally possible for two or more adjacent R substituents to form a monocyclic or polycyclic, aliphatic, aromatic or heteroaromatic ring system which may be substituted by one or more R¹ radicals;
R¹ is the same or different at each instance and is selected from the group consisting of H, D, F, Cl, Br, CN, a straight-chain alkyl or alkoxy group having 1 to 40 carbon atoms or a branched or cyclic alkyl or alkoxy group having 3 to 40 carbon atoms or an alkenyl group having 2 to 40 carbon atoms, each of which may be substituted by one or more R² radicals, where one or more hydrogen atoms may be replaced by D or F, an aromatic or heteroaromatic ring system which has 5 to 60 aromatic ring atoms and may be substituted in each case by one or more R² radicals;
Ar⁴ is the same or different at each instance and is an aromatic or heteroaromatic ring system which has 5-30 aromatic ring atoms and may be substituted by one or more nonaromatic R² radicals; at the same time, two Ar⁴ radicals bonded to the same nitrogen atom may also be bridged to one another by a single bond or a bridge selected from N(R²), C(R²)₂ and O;
R² is selected from the group consisting of H, D, F, CN or an aliphatic hydrocarbyl radical having 1 to 20 carbon atoms.

2. Compound according to Claim 1, **characterized in that** not more than one X group per cycle is N.

3. Compound according to Claim 1 or 2, selected from the compounds of the formulae (16) to (25) 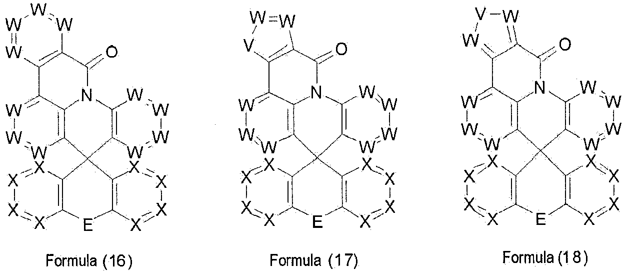 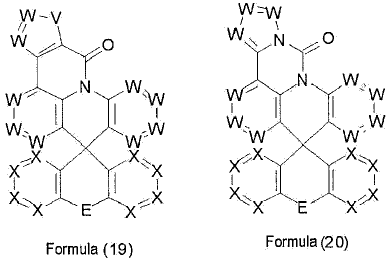 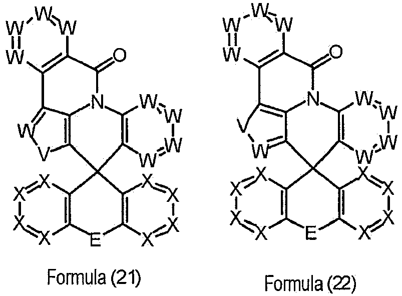 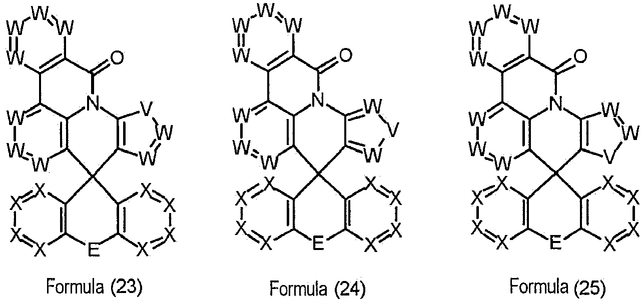 where the symbols used have the definitions given in Claim 1.

4. Compound according to one or more of Claims 1 to 3, selected from the compounds of the formulae (16a) to (25a) 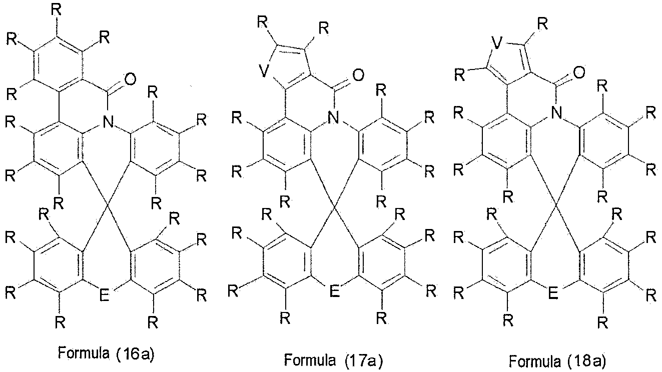 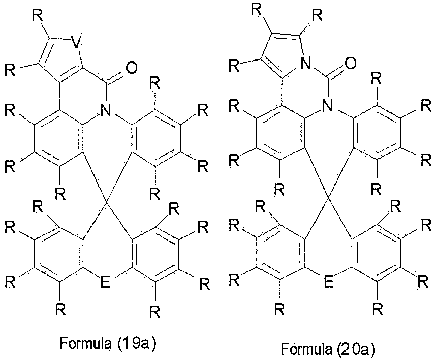 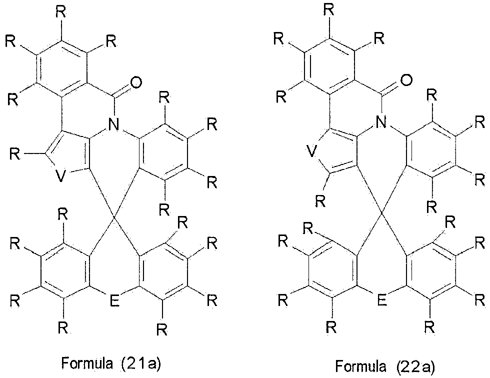 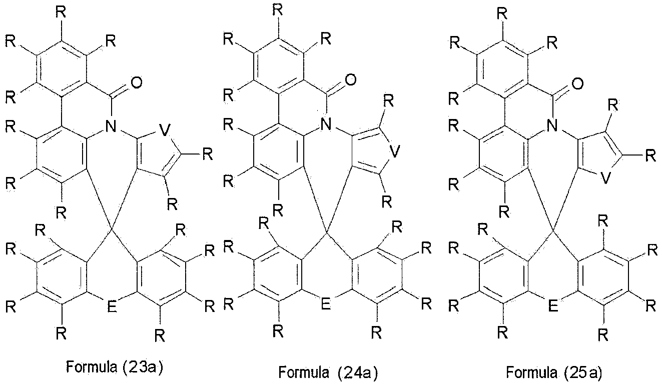 where the symbols used have the definitions given in Claim 1.

5. Compound according to one or more of Claims 1 to 4, selected from the compounds of the formulae (16b) to (25b) 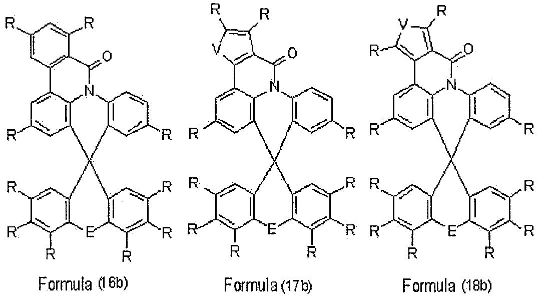 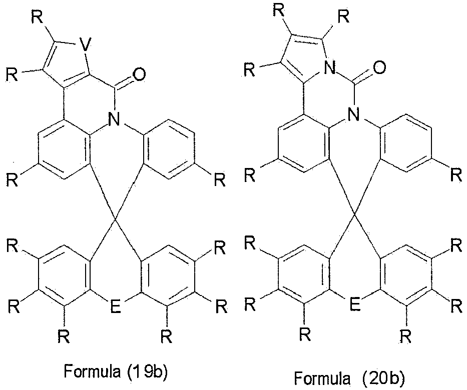 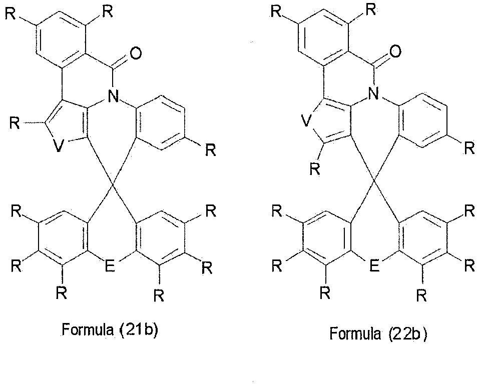 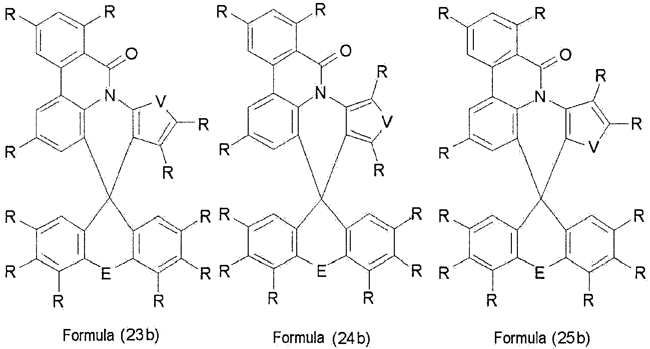 where the symbols used have the definitions given in Claim 1.

6. Compound according to one or more of Claims 1 to 5, **characterized in that** E is NR and R is selected from aromatic and heteroaromatic ring systems which have 5 to 40 aromatic ring atoms and may be substituted by one or more R¹ radicals, especially phenyl, ortho-, meta- or para-biphenyl, ortho-, meta-, para- or branched terphenyl, ortho-, meta-, para- or branched quaterphenyl, 1-, 2-, 3- or 4-fluorenyl, 1-, 2-, 3- or 4-spirobifluorenyl, triazine, 1-, 2-, 3- or 4-dibenzofuranyl, 1-, 2-, 3- or 4-dibenzothienyl or 1-, 2-, 3- or 4-carbazolyl, each of which may be substituted by one or more R¹ radicals.

7. Formulation, especially a solution or dispersion, comprising at least one compound according to one or more of Claims 1 to 6 and at least one further compound, especially a solvent.

8. Process for preparing a compound according to one or more of Claims 1 to 6, comprising the reaction steps of:
a) preparing a spiro compound proceeding from an acridine, xanthone, thioxanthenone or anthracen-9-one derivative; and
b) substituting the nitrogen, followed by cyclizing to give the lactam.

9. Use of a compound according to one or more of Claims 1 to 6 in an electronic device, especially in an organic electroluminescent device.

10. Electronic device, especially selected from the group consisting of organic electroluminescent devices, organic integrated circuits, organic field-effect transistors, organic thin-film transistors, organic light-emitting transistors, organic solar cells, organic dye-sensitized solar cells, organic optical detectors, organic photoreceptors, organic field-quench devices, light-emitting electrochemical cells, organic laser diodes and organic plasmon emitting devices, comprising one or more compounds according to one or more of Claims 1 to 6.

11. Electronic device according to Claim 10 which is an organic electroluminescent device, **characterized in that** the compound according to one or more of Claims 1 to 6 is used as matrix material for fluorescent or phosphorescent emitters and/or in a hole blocker layer and/or in an electron transport layer and/or in an electron-blocking or exciton-blocking layer and/or in a hole transport layer.

## Revendications

1. Composé selon la formule (1) 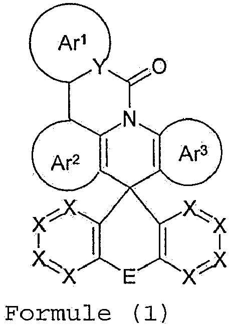 où, pour les symboles et indices utilisés, ce qui suit est d'application :
X représente en chaque occurrence, de manière identique ou différente, CR ou N ;
Y représente C, lorsque Ar¹ représente un groupe cyclique aryle ou hétéroaryle à 6 chaînons ou, selon le cas, C ou N, lorsque Ar¹ représente un groupe cyclique hétéroaryle à 5 chaînons ;
E représente NR, CR₂, O, S ou C=O ;
Ar¹ représente, ensemble avec le groupe Y et l'atome de carbone explicitement représenté, un groupe de formule (2), (3), (4), (5) ou (6), 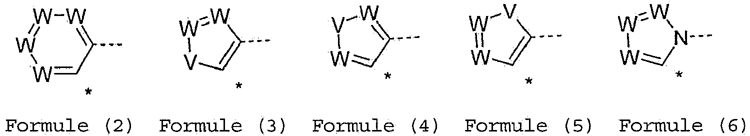
où la liaison en pointillés indique la liaison avec le groupe carbonyle du lactame, * indique la position de la liaison avec Ar² et en outre :
W représente en chaque occurrence, de manière identique ou différente, CR ou N ;
V représente NR, O ou S ;
Ar² représente, ensemble avec les atomes de carbone explicitement représentés, un groupe selon l'une des formules (9), (10) ou (11), 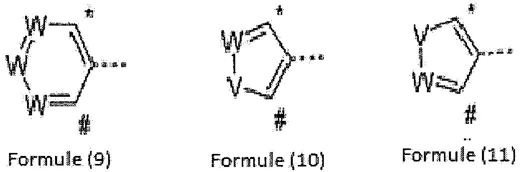 où la liaison en pointillés indique la liaison avec N, # indique la position de la liaison avec l'atome de carbone spiro, * indique la liaison avec Ar¹ et W et V présentent les significations susmentionnées,
Ar³ représente, ensemble avec les atomes de carbone explicitement représentés, un groupe selon l'une des formules (12), (13), (14) ou (15), 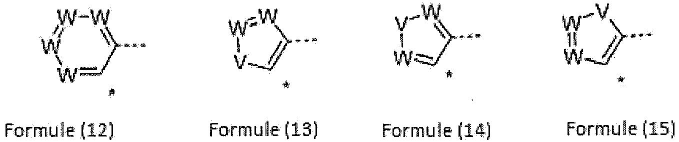 où la liaison en pointillés indique la liaison avec N, * indique la liaison avec l'atome de carbone spiro et W et V présentent les significations susmentionnées,
R en chaque occurrence, de manière identique ou différente, est choisi dans le groupe constitué par H, D, F, Cl, Br, CN, N(Ar⁴)₂, un groupe alkyle ou alcoxy linéaire comprenant 1 à 10 atomes de carbone ou un groupe alkyle ou alcoxy ramifié ou cyclique comprenant 3 à 10 atomes de carbone ou un groupe alcényle comprenant 2 à 10 atomes de carbone, qui peut à chaque fois être substitué par un ou plusieurs radicaux R¹, un ou plusieurs groupes CH2 non adjacents pouvant être remplacés par O et un ou plusieurs atomes de H pouvant être remplacés par D ou F, un système cyclique aromatique ou hétéroaromatique comprenant 5 à 30 atomes de cycle aromatique, qui peut à chaque fois être substitué par un ou plusieurs radicaux R¹, un groupe aryloxy ou hétéroaryloxy comprenant 5 à 30 atomes de cycle aromatique, qui peut être substitué par un ou plusieurs radicaux R¹, deux substituants R adjacents, ou plus, pouvant éventuellement former un système cyclique monocyclique ou polycyclique, aliphatique, aromatique et/ou hétéroaromatique, qui peut être substitué par un ou plusieurs radicaux R¹ ;
R¹ en chaque occurrence, de manière identique ou différente, est choisi dans le groupe constitué par H, D, F, Cl, Br, CN, un groupe alkyle ou alcoxy linéaire comprenant 1 à 40 atomes de carbone ou un groupe alkyle ou alcoxy ramifié ou cyclique comprenant 3 à 40 atomes de carbone ou un groupe alcényle comprenant 2 à 40 atomes de carbone, qui peut à chaque fois être substitué par un ou plusieurs radicaux R², un ou plusieurs atomes de H pouvant être remplacés par D ou F, un système cyclique aromatique ou hétéroaromatique comprenant 5 à 60 atomes de cycle aromatique, qui peut à chaque fois être substitué par un ou plusieurs radicaux R² ;
Ar⁴ en chaque occurrence, de manière identique ou différente, représente un système cyclique aromatique ou hétéroaromatique comprenant 5-30 atomes de cycle aromatique, qui peut être substitué par un ou plusieurs radicaux R² non aromatiques ; deux radicaux Ar⁴, qui se lient au même atome de N, pouvant également être pontés l'un à l'autre par l'intermédiaire d'une simple liaison ou d'un pont choisi parmi N(R²), C(R²)₂ ou O ;
R² est choisi dans le groupe constitué par H, D, F, CN ou un radical hydrocarboné aliphatique comprenant 1 à 20 atomes de carbone.

2. Composé selon la revendication 1, **caractérisé en ce qu'**au maximum un groupe X par cycle représente N.

3. Composé selon la revendication 1 ou 2, choisi parmi les composés des formules (16) à (25) 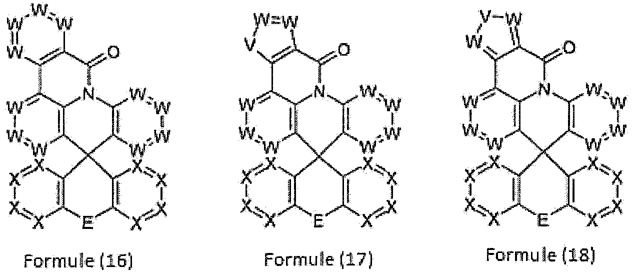 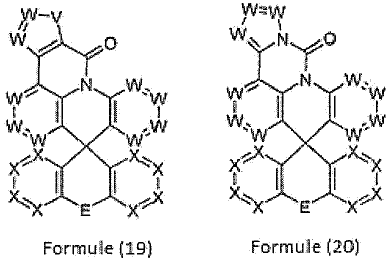 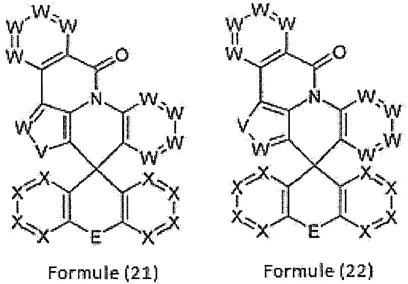 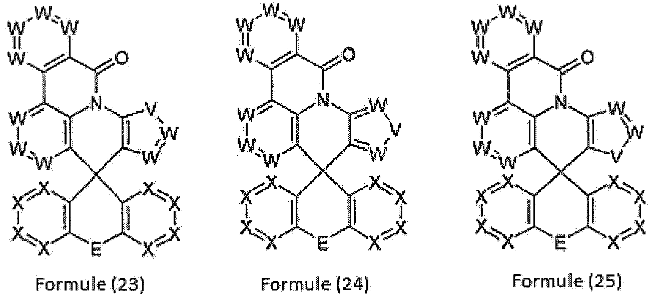 les symboles utilisés présentant les significations mentionnées dans la revendication 1.

4. Composé selon l'une ou plusieurs des revendications 1 à 3, choisi parmi les composés des formules (16a) à (25a) 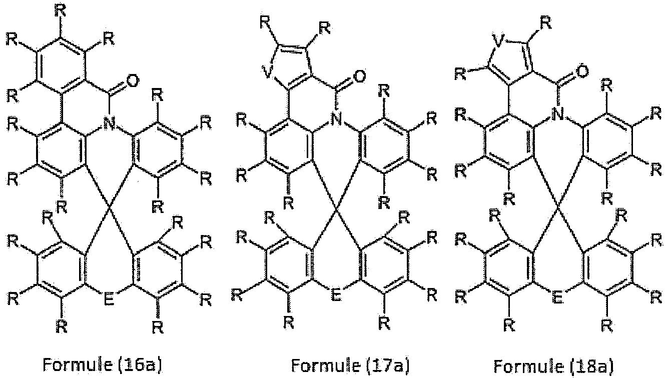 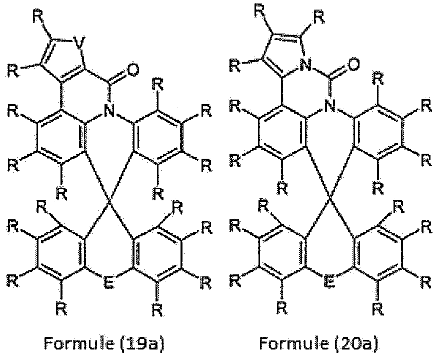 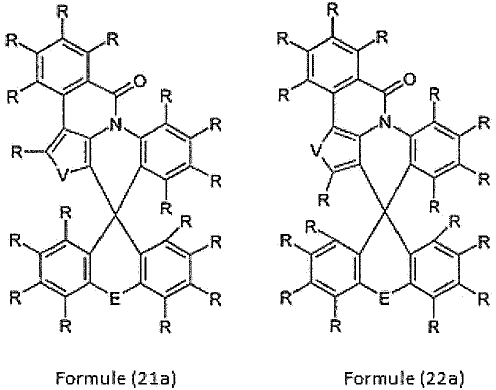 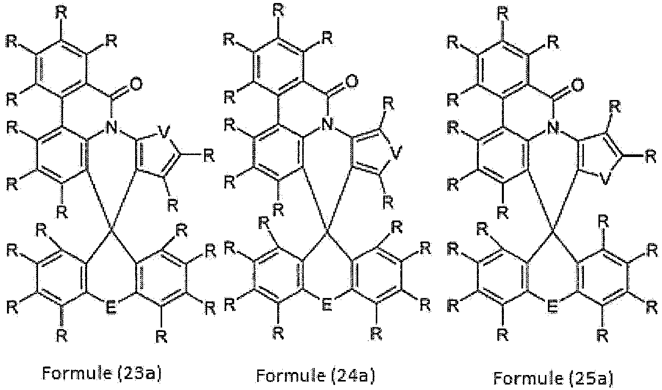 les symboles utilisés présentant les significations mentionnées dans la revendication 1.

5. Composé selon l'une ou plusieurs des revendications 1 à 4, choisi parmi les composés selon les formules (16b) à (25b) 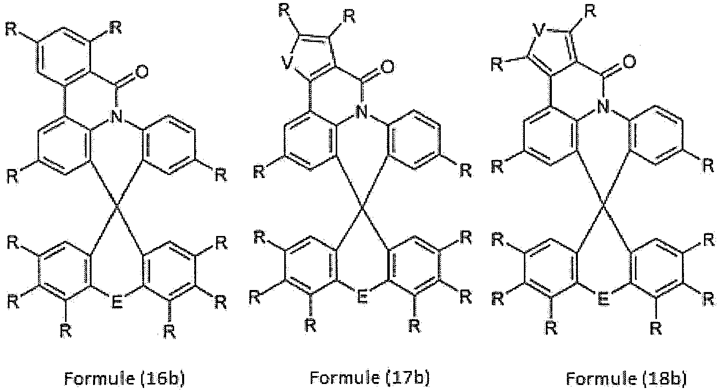 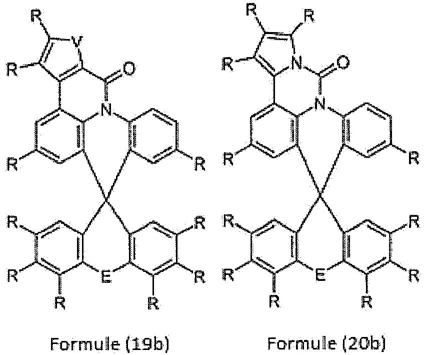 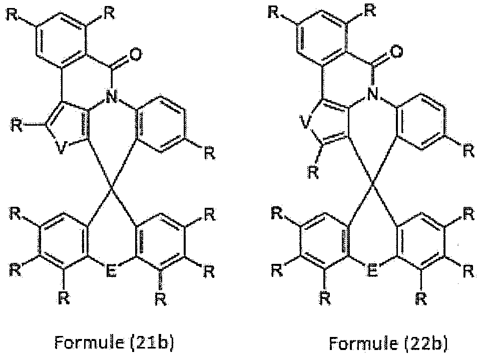 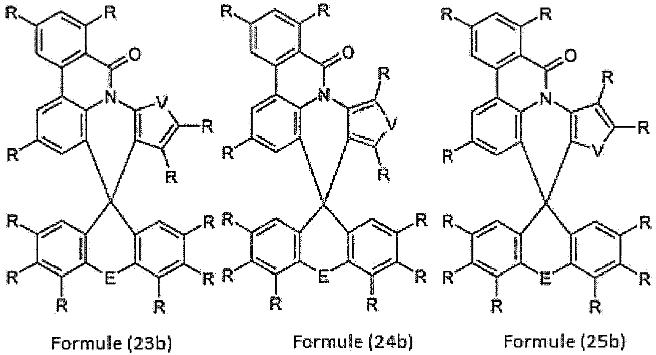 les symboles utilisés présentant les significations mentionnées dans la revendication 1.

6. Composé selon l'une ou plusieurs des revendications 1 à 5, **caractérisé en ce que** E représente NR et R est choisi parmi les systèmes cycliques aromatiques et hétéroaromatiques comprenant 5 à 40 atomes de cycle aromatique, qui peuvent être substitués par un ou plusieurs radicaux R¹, en particulier phényle, ortho-biphényle, méta-biphényle ou para-biphényle, ortho-terphényle, méta-terphényle, para-terphényle ou terphényle ramifié, ortho-quaterphényle, méta-quaterphényle, para-quaterphényle ou quaterphényle ramifié, 1-fluorényle, 2-fluorényle, 3-fluorényle ou 4-fluorényle, 1-spirobifluorényle, 2-spirobifluorényle, 3-spirobifluorényle ou 4-spirobifluorényle, triazine, 1-dibenzofurannyle, 2-dibenzofurannyle, 3-dibenzofurannyle ou 4-dibenzofurannyle, 1-dibenzothiényle, 2-dibenzothiényle, 3-dibenzothiényle ou 4-dibenzothiényle ou 1-carbazolyle, 2-carbazolyle, 3-carbazolyle ou 4-carbazolyle, qui peuvent à chaque fois être substitués par un ou plusieurs radicaux R¹.

7. Formulation, en particulier solution ou dispersion, contenant au moins un composé selon l'une ou plusieurs des revendications 1 à 6 et au moins un autre composé, en particulier un solvant.

8. Procédé pour la préparation d'un composé selon l'une ou plusieurs des revendications 1 à 6, comprenant les étapes de réaction :
a) préparation d'un composé spiro partant d'un dérivé d'acridine, de xanthone, de thioxanthénone ou d'anthracén-9-one ; et
b) substitution de l'azote suivie d'une cyclisation en lactame.

9. Utilisation d'un composé selon l'une ou plusieurs des revendications 1 à 6 dans un dispositif électronique, en particulier un dispositif organique électroluminescent.

10. Dispositif électronique, en particulier choisi dans le groupe constitué par les dispositifs organiques électroluminescents, les circuits organiques intégrés, les transistors organiques à effet de champ, les transistors organiques à film mince, les transistors organiques électroluminescents, les piles solaires organiques, les piles solaires organiques sensibilisées par un colorant, les détecteurs organiques optiques, les photorécepteurs organiques, les dispositifs organiques à extinction de champ, les piles électrochimiques électroluminescentes, les diodes laser organiques et les dispositifs organiques émettant un plasmon ("organic plasmon emitting devices") contenant un ou plusieurs composés selon l'une ou plusieurs des revendications 1 à 6.

11. Dispositif électronique selon la revendication 10, le dispositif étant un dispositif organique électroluminescent, **caractérisé en ce que** le composé selon l'une ou plusieurs des revendications 1 à 6 est utilisé comme matériau de matrice pour des émetteurs fluorescents ou phosphorescents et/ou dans une couche de blocage de trous et/ou dans une couche de transport d'électrons et/ou dans une couche de blocage d'électrons ou de blocage d'excitons et/ou dans une couche de transport de trous.
